(19) 

(11) **EP 4 251 246 B1**

(12) **EUROPÄISCHE PATENTSCHRIFT**

(45) Veröffentlichungstag und Bekanntmachung des Hinweises auf die Patenterteilung:
**23.09.2026 Patentblatt 2026/39**

(21) Anmeldenummer: **21810534.4**

(22) Anmeldetag: **19.11.2021**

(51) Internationale Patentklassifikation (IPC):
***A61M 16/00*** *(2006.01)*

(52) Gemeinsame Patentklassifikation (CPC):
**A61M 16/026;** A61M 16/0069; A61M 2016/0027; A61M 2016/0036; A61M 2205/18; A61M 2205/502; A61M 2205/70; A61M 2230/42; A61M 2230/46

(86) Internationale Anmeldenummer:
**PCT/EP2021/025452**

(87) Internationale Veröffentlichungsnummer:
**WO 2022/111848 (02.06.2022 Gazette 2022/22)**

(54) **ERKENNUNG VON ASYNCHRONIEN BEI DER BEATMUNG**

DETECTION OF ASYNCHRONY DURING RESPIRATORY TREATMENT

DÉTECTION D'ASYNCHRONISME PENDANT UN TRAITEMENT RESPIRATOIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.11.2020 DE 102020007180**
**07.08.2021 DE 102021004081**

(43) Veröffentlichungstag der Anmeldung:
**04.10.2023 Patentblatt 2023/40**

(60) Teilanmeldung:
**26152098.5 / 4 706 715**

(73) Patentinhaber: **Löwenstein Medical Technology S.A.**
**2557 Luxembourg (LU)**

(72) Erfinder:
- **VERHOEVEN, Jan**
  **76275 Ettlingen (DE)**
- **SCHWAIBOLD, Matthias**
  **76228 Karlsruhe (DE)**

(74) Vertreter: **Löwenstein Medical IP**
**Löwenstein Medical Technology GmbH + Co.KG IP Management**
**Kronsaalsweg 40**
**22525 Hamburg (DE)**

(56) Entgegenhaltungen:
**WO-A1-2019/094736**
**US-A1- 2012 037 159**
**US-A1- 2015 202 394**
**US-A1- 2016 325 061**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Processed by Luminess, 75001 PARIS (FR)

## Beschreibung

## Beschreibung

Die Erfindung betrifft ein System zur Erkennung von Asynchronien zwischen einem Beatmungsgerät und einem mit dem Beatmungsgerät verbundenen Lebewesen sowie zu einer damit zusammenhängenden, teilweise automatischen Steuerung eines Beatmungsgerätes. Menschen atmen etwa 20000-mal am Tag ein und aus. Für die Steuerung eines Beatmungsgerätes ist es eine nicht unerhebliche Aufgabe, diese Atemzüge korrekt zu erkennen und eine Atemunterstützung zum richtigen Zeitpunkt auszulösen. Präzise Algorithmen zur Auslösung der Atemzüge sind dabei ausschlaggebend für den Erfolg der Therapie und das Wohlbefinden des Patienten oder Anwenders. Häufig wird die Empfindlichkeit für die Auslösung der Atemzüge von einem behandelnden Arzt oder Betreuer manuell eingestellt. Die so gewählten Einstellungen können aber zum Teil nicht optimal an die individuellen Bedürfnisse der Patienten während der Therapie angepasst sein, da sich durch Positionswechsel, verschiedene Schlafphasen etc. die nötige Auslöseempfindlichkeit ändert oder ändern kann.

US 2016/325061 A1 offenbart beispielsweise ein System zur nicht invasiven Erkennung einer Asynchronität zwischen einem Patienten und einem Beatmungsgerät. Dabei kann eine Variabilität einer oder mehrerer Beatmungsparameter über mehrere Atemzyklen hinweg bestimmt werden. Anhand der Variabilität kann dann die Asynchronität erkannt werden. Aufgabe der vorliegenden Erfindung ist daher die Bereitstellung eines Systems zur effektiven und sicheren Beatmung eines Lebewesens. Die Aufgabe wird durch das System gemäß Anspruch 1 gelöst. Bevorzugte Ausführungsformen der Erfindung sind Gegenstand der abhängigen Ansprüche.

Offenbart wird ein System zur Erkennung von Asynchronien zwischen einem Beatmungsgerät und einem Lebewesen, umfassend zumindest ein Beatmungsgerät, wobei das zumindest eine Beatmungsgerät zumindest

- eine Sensoreinheit
- eine Aufbereitungseinheit
- eine Berechnungseinheit
- eine Erkennungseinheit
- eine Speichereinheit
- eine Überwachungseinheit
- eine Steuereinheit und
- eine Gebläse-/Ventileinheit

umfasst, wobei die Erkennungseinheit anhand von Atemparametern des Lebewesens Asynchronien zwischen dem Beatmungsgerät und dem Lebewesen erkennt.

In manchen Ausführungsformen des Systems enthalten die Atemparameter zumindest Druck und/oder Fluss und/oder werden aus diesen bestimmt.

In manchen Ausführungsformen des Systems steuert die Steuereinheit anhand der durch die der Erkennungseinheit erkannten Asynchronien die Gebläse-/Ventileinheit.

In manchen Ausführungsformen des Systems wird die Erkennung der Asynchronien während der Verwendung des Beatmungsgerätes durch das Lebewesen durchgeführt. Die Asynchronieerkennung wird also durchgeführt, während das Lebewesen das Beatmungsgerät verwendet.

In manchen Ausführungsformen des Systems erkennt die Erkennungseinheit verpasste Atemzüge und kurze Auslöseverzögerungen.

In manchen Ausführungsformen des Systems bewertet die Erkennungseinheit die kurzen Auslöseverzögerungen und die verpassten Atemzüge als Asynchronien.

In manchen Ausführungsformen des Systems erkennt die Erkennungseinheit die verpassten Atemzüge durch Auswertung eines Atemanstrengungsflusses, eines erwarteten Atemflusses sowie eines bestimmten Atemflusses.

In manchen Ausführungsformen des Systems erkennt die Erkennungseinheit die kurzen Auslöseverzögerungen durch Auswertung eines Atemanstrengungsflusses, eines erwarteten Atemflusses sowie eines bestimmten Atemflusses.

In manchen Ausführungsformen des Systems erkennt die Erkennungseinheit falsche Auslösungen und bewertet diese als Asynchronie.

In manchen Ausführungsformen des Systems bestimmt die Berechnungseinheit den Atemanstrengungsfluss aus dem erwarteten Atemfluss und dem bestimmten Atemfluss.

In manchen Ausführungsformen des Systems bestimmt die Berechnungseinheit den erwarteten Atemfluss aus dem Atemwegswiderstand R und der Lungenelastizität E.

In manchen Ausführungsformen des Systems bestimmt die Berechnungseinheit den erwarteten Atemfluss aus einem Mittelwert des Atemwegswiderstandes R und einem Mittelwert der Lungenelastizität E.

In manchen Ausführungsformen des Systems berechnet die Berechnungseinheit den Atemwegswiderstand R und die Lungenelastizität E aus den durch die Sensoreinheit gemessenen und durch die Aufbereitungseinheit aufbereiteten Messwerten.

In manchen Ausführungsformen des Systems stellt die Steuereinheit anhand der von der Erkennungseinheit erkannten verpassten Atemzüge und kurzer Auslöseverzögerungen die Auslöseempfindlichkeit des Beatmungsgerätes automatisch ein.

In manchen Ausführungsformen des Systems stellt die Steuereinheit anhand der von der Erkennungseinheit erkannten verpassten Atemzüge, kurzer Auslöseverzögerungen und falscher Auslösungen die Auslöseempfindlichkeit des Beatmungsgerätes automatisch ein.

In manchen Ausführungsformen des Systems werden der Atemwegswiderstand R und die Lungenelastizität E durch die Berechnungseinheit über ein mathematisches Lungenmodell bestimmt.

In manchen Ausführungsformen des Systems werden der Atemwegswiderstand R und die Lungenelastizität E durch die Berechnungseinheit über eine multiple lineare Regression und das Ein-Kompartment-Lungenmodell bestimmt.

Erfindungsgemäß erkennt die Erkennungseinheit die verpassten Atemzüge anhand von zumindest zwei der folgenden Merkmale des Atemanstrengungsflusses, des erwarteten Atemflusses und des bestimmten Atemflusses:

- das lokale Maximum des bestimmten Atemflusses liegt zwischen zwei Minima des Atemanstrengungsflusses;
- Differenz zwischen der zeitlichen Lage des lokalen Maximums des Atemanstrengungsflusses und dem entsprechenden linken Minimum;
- Differenz zwischen der zeitlichen Lage des lokalen Maximums des Atemanstrengungsflusses und dem entsprechenden rechten Minimum;
- Differenz zwischen den Werten des lokalen Maximums des Atemanstrengungsflusses und dem entsprechenden linken Minimum;
- Differenz zwischen den Werten des lokalen Maximums des Atemanstrengungsflusses und dem entsprechenden rechten Minimum;
- erwarteter Atemfluss zum Zeitpunkt des lokalen Maximums des Atemanstrengungsflusses;
- Zeit zwischen dem lokalen Maximum des Atemanstrengungsflusses und dem erwarteten Auslösezeitpunkt.

In manchen Ausführungsformen des Systems erkennt die Erkennungseinheit die verpassten Atemzüge anhand folgender Merkmale des Atemanstrengungsflusses, des erwarteten Atemflusses und des bestimmten Atemflusses:

- das lokale Maximum des bestimmten Atemflusses muss zwischen zwei Minima des Atemanstrengungsflusses liegen;
- Differenz zwischen der zeitlichen Lage des lokalen Maximums des Atemanstrengungsflusses und dem entsprechenden linken Minimum;
- Differenz zwischen der zeitlichen Lage des lokalen Maximums des Atemanstrengungsflusses und dem entsprechenden rechten Minimum;
- Differenz zwischen den Werten des lokalen Maximums des Atemanstrengungsflusses und dem entsprechenden linken Minimum;
- Differenz zwischen den Werten des lokalen Maximums des Atemanstrengungsflusses und dem entsprechenden rechten Minimum;
- erwarteter Atemfluss zum Zeitpunkt des lokalen Maximums des Atemanstrengungsflusses;
- Zeit zwischen dem lokalen Maximum des Atemanstrengungsflusses und dem erwarteten Auslösezeitpunkt.

In manchen Ausführungsformen des Systems berechnet die Berechnungseinheit den erwarteten Auslösezeitpunkt über einen Mittelwert der letzten Atemzuglängen.

In manchen Ausführungsformen des Systems berechnet die Berechnungseinheit den erwarteten Auslösezeitpunkt über einen Mittelwert der Länge des letzten spontanen Atemzuges sowie der letzten erwarteten Auslösezeit.

In manchen Ausführungsformen des Systems ist der Mittelwert ein gewichteter Mittelwert.

In manchen Ausführungsformen des Systems erkennt die Erkennungseinheit anhand des von der Berechnungseinheit bestimmten Wertes der Auslöseverzögerung, ob es sich um eine kurze Auslöseverzögerung handelt.

In manchen Ausführungsformen des Systems erkennt und bestimmt die Erkennungseinheit die Auslöseverzögerung über den Versatz zwischen der Atemanstrengung des Lebewesens und der Auslösung des Beatmungsgerätes.

In manchen Ausführungsformen des Systems erkennt die Erkennungseinheit kurze Auslöseverzögerungen daran, dass die Auslöseverzögerung als zu kurz für eine Atemanstrengung durch das Lebewesen bewertet wird.

In manchen Ausführungsformen des Systems wird eine Auslöseverzögerung als kurze Auslöseverzögerung erkannt, wenn die Auslöseverzögerung kleiner oder gleich einem Schwellenwert ist, wobei dieser Schwellenwert in einem Bereich zwischen 0 Sekunden und 0,5 Sekunden, bevorzugt zwischen 0 Sekunden und 0,25 Sekunden, besonders

bevorzugt zwischen 0 Sekunden und 0,15 Sekunden, gewählt wird.

In manchen Ausführungsformen des Systems ist der Schwellenwert zur Erkennung einer kurzen Auslöseverzögerung 0,1 Sekunden.

In manchen Ausführungsformen des Systems werden vom Beatmungsgerät vorgegebene Atemzüge nicht auf Auslöseverzögerungen untersucht, wobei vom Beatmungsgerät vorgegebene Atemzüge nicht durch eine Atemanstrengung des Lebewesens ausgelöst werden.

In manchen Ausführungsformen des Systems passt die Steuereinheit die Auslöseempfindlichkeit entsprechend der Anzahl von erkannten kurzen Auslöseverzögerungen und verpassten Atemzügen innerhalb eines Zeitintervalls automatisch an.

In manchen Ausführungsformen des Systems beträgt das Zeitintervall zwischen 0,5 und 5 Minuten, bevorzugt zwischen 1 und 3 Minuten.

In manchen Ausführungsformen des Systems wird bei Leckageflüssen über einem Schwellenwert zwischen 15 l/min und 50 l/min die Auslöseempfindlichkeit in Form einer durchschnittlichen Auslöseempfindlichkeit eingestellt, welche unter Einbeziehung von Auslöseempfindlichkeiten aus vorherigen Zeiträumen mit Leckageflüssen unter dem Schwellenwert bestimmt wird.

In manchen Ausführungsformen des Systems wird bei Leckageflüssen von über 25 l/min die Auslöseempfindlichkeit in Form einer durchschnittlichen Auslöseempfindlichkeit eingestellt, welche unter Einbeziehung von Auslöseempfindlichkeiten aus vorherigen Zeiträumen mit Leckageflüssen unter 25 l/min bestimmt wird.

In manchen Ausführungsformen des Systems beschreibt die Auslöseempfindlichkeit die Parameter, nach denen das Beatmungsgerät einen Atemzug des Lebewesens erkennt und eine Unterstützung der Beatmung auslöst, wobei die Parameter zumindest einen Schwellenwert des Atemflusses umfassen.

In manchen Ausführungsformen des Systems ist die Auslöseempfindlichkeit manuell und automatisch einstellbar.

In manchen Ausführungsformen des Systems kann die automatische Einstellung der Auslöseempfindlichkeit geringere Schwellenwerte der Parameter zur Auslösung einstellen, als durch die manuelle Einstellung möglich ist.

In manchen Ausführungsformen des Systems werden die Parameter zur Einstellung der Auslöseempfindlichkeit in abstrakten, einheitslosen Zahlenwerten zusammengefasst, wobei die Zahlenwerte von 0 bis 8 reichen und wobei ein niedriger Zahlenwert eine hohe Auslöseempfindlichkeit und ein hoher Zahlenwert eine niedrige Auslöseempfindlichkeit wiedergibt.

In manchen Ausführungsformen des Systems stehen für die automatische Einstellung der Auslöseempfindlichkeit Werte von 0 bis 3 und für die manuelle Einstellung Werte von 1 bis 8 zur Verfügung.

In manchen Ausführungsformen des Systems werden die Stufen der Auslöseempfindlichkeit zumindest anhand von Schwellenwerten des Atemflusses definiert.

In manchen Ausführungsformen des Systems ist das Beatmungsgerät ausgebildet, um anhand der erkannten verpassten Atemzüge und/oder kurzen Auslöseverzögerung eine Empfehlung zur manuellen Einstellung der Auslöseempfindlichkeit bereitzustellen.

In manchen Ausführungsformen des Systems ist das Beatmungsgerät ausgebildet, um bei der Überschreitung eines Schwellenwertes an verpassten Atemzügen und/oder kurzen Auslöseverzögerungen einen Alarm zu generieren.

In manchen Ausführungsformen des Systems ist das Beatmungsgerät ausgebildet, um zumindest die durch die Erkennungseinheit erkannten verpassten Atemzüge und/oder kurzen Auslöseverzögerungen zu verwenden, um ungünstige Einstellungen des Beatmungsgerätes zu erkennen.

In manchen Ausführungsformen des Systems ist das Beatmungsgerät ausgebildet, um zumindest die durch die Erkennungseinheit erkannten verpassten Atemzüge und/oder kurzen Auslöseverzögerungen zu verwenden, um einen intrinsischen PEEP zu erkennen.

In manchen Ausführungsformen des Systems wird der Atemanstrengungsfluss durch einen Tiefpass-Filter gefiltert und die Berechnungseinheit berechnet unter Verwendung des gefilterten Atemanstrengungsflusses die Auslöseverzögerung.

In manchen Ausführungsformen des Systems ist das Beatmungsgerät ausgebildet, um verfrühte Exspirationstrigger zu erkennen.

In manchen Ausführungsformen des Systems ist das Beatmungsgerät ausgebildet, um verfrühte Exspirationstrigger als Asynchronie zu bewerten.

In manchen Ausführungsformen des Systems ist das Beatmungsgerät ausgebildet, um verfrühte Exspirationstrigger anhand des zeitlichen Verlaufs des bestimmten Atemflusses und/oder des erwarteten Atemflusses und/oder des Atemanstrengungsflusses zu erkennen.

In manchen Ausführungsformen des Systems ist das Beatmungsgerät ausgebildet, um verfrühte Exspirationstrigger anhand der Lage und des Wertes der Flussrate eines Starts einer Druckrampe, eines Endes einer Druckrampe, eines lokalen Maximums und/oder eines lokalen Minimums des bestimmten Atemflusses und/oder des erwarteten Atemflusses zu erkennen.

In manchen Ausführungsformen des Systems ist das Beatmungsgerät ausgebildet, um verfrühte Exspirationstrigger anhand zumindest eines der folgenden Punkte zu erkennen:

- der bestimmte Atemfluss liegt zu Beginn der Druckrampe über dem Wert des bestimmten Atemflusses zum Ende der Druckrampe;
- der bestimmte Atemfluss liegt zu Beginn der Druckrampe über einem Wert von $q_1$;
- der bestimmte Atemfluss liegt zum Ende der Druckrampe unter einem Wert von $q_2$;
- e * bestimmter Atemfluss (am lokalen Maximum) / bestimmter Atemfluss (zum Ende der Druckrampe) < erwarteter Atemfluss (Maximum) / erwarteter Atemfluss (zum Ende der Druckrampe);
- f * bestimmter Atemfluss (am lokalen Maximum) > bestimmter Atemfluss (zum Ende der Druckrampe);
- g * bestimmter Atemfluss (am lokalen Minimum) < bestimmter Atemfluss (zum Ende der Druckrampe) + bestimmter Atemfluss (am lokalen Maximum);
- (h < bestimmter Atemfluss (am lokalen Maximum) - bestimmter Atemfluss (zum Ende der Druckrampe)) ODER (h < bestimmter Atemfluss (lokales Maximum) - bestimmter Atemfluss (am lokalen Minimum))
- Bestimmter Atemfluss (am lokalen Minimum) < $q_3$.

Dabei liegt der Faktor e in einem Bereich von 0,5 bis 2,0, bevorzugt zwischen 1 und 1,8, der Faktor f in einem Bereich von 0,9 bis 2,9, bevorzugt zwischen 1,7 und 2,3, der Faktor g in einem Bereich von 1 bis 3, bevorzugt zwischen 1,8 und 2,2, und h in einem Bereich von 2 l/min bis 10 l/min, bevorzugt zwischen 3 l/min und 7 l/min. Die Werte von $q_1$, $q_2$ und $q_3$ liegen beispielsweise unabhängig voneinander in einem Bereich von -5 l/min bis +5 l/min, bevorzugt zwischen -1 l/min und +1 l/min.

In manchen Ausführungsformen des Systems ist das Beatmungsgerät ausgebildet, um verfrühte Exspirationstrigger anhand folgender Punkte zu erkennen:

- der bestimmte Atemfluss liegt zu Beginn der Druckrampe über 0 l/min;
- der bestimmte Atemfluss liegt zum Ende der Druckrampe unter 0 l/min;
- e * bestimmter Atemfluss (am lokalen Maximum) / bestimmter Atemfluss (zum Ende der Druckrampe) < erwarteter Atemfluss (Maximum) / erwarteter Atemfluss (zum Ende der Druckrampe);
- f * bestimmter Atemfluss (am lokalen Maximum) > bestimmter Atemfluss (zum Ende der Druckrampe);
- g * bestimmter Atemfluss (am lokalen Minimum) < bestimmter Atemfluss (zum Ende der Druckrampe) + bestimmter Atemfluss (am lokalen Maximum);
- (h < bestimmter Atemfluss (am lokalen Maximum) - bestimmter Atemfluss (zum Ende der Druckrampe)) ODER (h < bestimmter Atemfluss (lokales Maximum) - bestimmter Atemfluss (am lokalen Minimum));
- bestimmter Atemfluss (am lokalen Minimum) < 0.

Dabei liegt der Faktor e in einem Bereich von 0,5 bis 2,0, bevorzugt zwischen 1 und 1,8, der Faktor f in einem Bereich von 0,9 bis 2,9, bevorzugt zwischen 1,7 und 2,3, der Faktor g in einem Bereich von 1 bis 3, bevorzugt zwischen 1,8 und 2,2, und h in einem Bereich von 2 l/min bis 10 l/min, bevorzugt zwischen 3 l/min und 7 l/min.

In manchen Ausführungsformen des Systems umfasst die Auslöseempfindlichkeit einen Wert, welcher eine Umschaltung von einer Inspirationsphase auf eine Exspirationsphase steuert, wobei das Beatmungsgerät ausgebildet ist, um anhand von erkannten verfrühten Exspirationstriggern den Wert der Auslöseempfindlichkeit, welcher die Umschaltung von einer Inspirationsphase auf eine Exspirationsphase steuert, einzustellen.

In manchen Ausführungsformen des Systems kann vorgesehen sein, dass die Erkennung von Asynchronien, beispielsweise verpassten Atemzügen, kurzen Auslöseverzögerungen und/oder verfrühten Exspirationstriggern, durch die Verwendung von Parametern wie (Atem-)Volumen, Druck, Fluss und/oder (Atem-)Frequenz zusätzlich verfeinert wird und/oder alternativ durchgeführt wird. Es kann beispielsweise vorgesehen sein, dass Merkmale des zeitlichen Verlaufs des Drucks, Flusses, der Atemfrequenz und/oder des Atemvolumens mit in die Erkennung der Asynchronien einbezogen werden.

Offenbart wird auch ein (nicht beanspruchtes) Verfahren zur Erkennung von Asynchronien zwischen einem Beatmungsgerät und einem Lebewesen, wobei die Asynchronien in Form von kurzen Auslöseverzögerungen und verpassten Atemzügen unter Verwendung der Lungenelastizität E und des Atemwegswiderstandes R des Lebewesens erkannt werden.

Es wird darauf hingewiesen, dass die in den Ansprüchen einzeln aufgeführten Merkmale in beliebiger, technisch sinnvoller Weise miteinander kombiniert werden können und weitere Ausgestaltungen der Erfindung aufzeigen. Die Beschreibung charakterisiert und spezifiziert die Erfindung insbesondere im Zusammenhang mit den Figuren zusätzlich.

Es wird ferner darauf hingewiesen, dass eine hierin verwendete, zwischen zwei Merkmalen stehende und diese miteinander verknüpfende "und/oder"-Konjunktion stets so auszulegen ist, dass in einer ersten Ausgestaltung lediglich das erste Merkmal vorhanden sein kann, in einer zweiten Ausgestaltung lediglich das zweite Merkmal vorhanden sein

kann und in einer dritten Ausgestaltung sowohl das erste als auch das zweite Merkmal vorhanden sein können.

Unter einem Beatmungsgerät ist jedwedes Gerät zu verstehen, welches einen Anwender oder Patienten bei der natürlichen Atmung unterstützt, die Beatmung des Anwenders bzw. Patienten übernimmt und/oder zur Atemtherapie dient und/oder anderweitig die Atmung des Anwenders bzw. Patienten beeinflusst. Darunter fallen zum Beispiel, aber nicht ausschließend, CPAP- sowie BiLevel-Geräte (teilweise bekannt als BiPAP), Narkose- bzw. Anästhesiegeräte, Atemtherapiegeräte, (klinische, außerklinische oder Notfall-)Beatmungsgeräte, Highflow-Therapiegeräte und Hustenmaschinen. Beatmungsgeräte können auch als Diagnosegeräte für die Beatmung verstanden werden. Diagnosegeräte können dabei allgemein zur Erfassung von medizinischen Parametern eines Patienten dienen. Darunter fallen auch Geräte, welche medizinische Parameter von Patienten in Kombination mit oder ausschließlich die Atmung betreffend, erfassen und optional verarbeiten können.

Als Patienteninterface kann, soweit nicht ausdrücklich anders beschrieben, jeglicher Teil (oder verbundene Peripheriegeräte) des Beatmungsgerätes verstanden werden, welcher zur Interaktion, insbesondere zu Therapie- oder Diagnosezwecken, mit einem Patienten gedacht ist. Insbesondere kann ein Patienteninterface als Maske eines Beatmungsgerätes oder als eine mit dem Beatmungsgerät verbundene Maske verstanden werden. Diese Maske kann eine Full-Face-Maske, also eine die Nase und den Mund umschließende Maske, oder eine Nasenmaske, also eine nur die Nase umschließende Maske, sein. Auch Trachealtuben und sogenannte Nasenbrillen können als Masken eingesetzt werden.

Das System eignet sich insbesondere zur Anwendung im Bereich der Therapie und Beatmung von Patienten. Das System eignet sich darüber hinaus auch zur Anwendung in anderen Bereichen, bei denen eine Unterstützung der natürlichen Atmung gewünscht sein kann, wie zum Beispiel bei Tauchern, Bergsteigern, in der Schutzausrüstung von Einsatzkräften der Feuerwehr etc. Zudem können das System und das Verfahren auch Anwendung bei nicht menschlichen Lebewesen finden. Es wird daher darauf hingewiesen, dass die beschriebenen Ausführungsbeispiele auf Menschen ausgelegt sind und bei Ausführungsformen für nicht menschliche Lebewesen gegebenenfalls Parameter wie zum Beispiel Fluss- und Volumengrößen und auch Zeitspannen, beispielsweise Atemzuglängen, angepasst werden müssen.

Unter Asynchronien sind Abweichungen der vorgegebenen Atemcharakteristiken des Beatmungsgeräts und der natürlichen und/oder gewollten Atmung des Lebewesens zu verstehen. Beispielsweise ist es als Asynchronie zu verstehen, wenn das Lebewesen eine Anstrengung, einzuatmen, zeigt, das Beatmungsgerät diese Anstrengung nicht erkennt und entsprechend keine Unterstützung zur Atmung, insbesondere zur Inspiration, auslöst. Auch ist unter einer Asynchronie zu verstehen, dass ein Beatmungsgerät einen Atemzug erkennt und entsprechend eine Unterstützung oder Beatmung auslöst, obwohl das Lebewesen keinen Atemzug beabsichtigt hat. In manchen Ausführungsformen ist das Beatmungsgerät so eingestellt, dass von Zeit zu Zeit das Beatmungsgerät eine Beatmung auslöst, ohne dass eine Atemanstrengung des Lebewesens erkannt wird. Dies ist beispielsweise der Fall, wenn das Beatmungsgerät über einen Zeitraum keine Atemanstrengung des Lebewesens erkannt hat. Solche mandatorischen oder erzwungenen Beatmungen werden hingegen nicht als Asynchronien verstanden. In manchen Ausführungsformen können auch weitere Abweichungen, beispielsweise eine Phasenverschiebung (Patient atmet aus, Beatmungsgerät erkennt aber ein Einatmen) oder zeitversetzte Unterstützung (Beatmungsgerät reagiert zu spät auf Atemanstrengungen oder verpasst den Übergang von der Inspiration zur Exspiration) als Asynchronien verstanden werden.

Es kann zwischen zumindest drei Arten von Atemzügen unterschieden werden: spontanen Atemzügen, verpassten Atemzügen und durch das Beatmungsgerät vorgegebenen Atemzügen. Als spontane Atemzüge sind jene Atemzüge anzusehen, welche durch das Beatmungsgerät erkannt und entsprechend zumindest in der inspiratorischen Phase, also dem Einatmen, durch das Beatmungsgerät unterstützt werden. Verpasste Atemzüge sind jene Atemzüge, bei denen das Lebewesen zwar eine Atemanstrengung zeigt, diese aber nicht durch das Beatmungsgerät erkannt wird und daher auch keine inspiratorische Unterstützung erfolgt. Vorgegebene Atemzüge sind Atemzüge, welche durch das Beatmungsgerät vorgegeben werden, ohne dass das Lebewesen eine Atemanstrengung zeigt oder eine Atemanstrengung durch das Beatmungsgerät erkannt wird. Ein vorgegebener Atemzug findet beispielsweise statt, wenn ein Zeitraum nach dem letzten erkannten Atemzug überschritten wird, ohne dass ein weiterer Atemzug erfolgte.

Es wird darauf hingewiesen, dass Auslöseverzögerungen für jede Auslösung der inspiratorischen Unterstützung des Beatmungsgerätes bestimmt werden - sowohl für spontane als auch für vorgegebene Atemzüge. Eine Auslöseverzögerung allein stellt für das System noch keine Asynchronie dar. Lediglich solche Auslöseverzögerungen, welche unter einem bestimmten Schwellenwert liegen, also kurze Auslöseverzögerungen sind, werden als Asynchronie bewertet. Kurze Auslöseverzögerungen sind daher als interessant anzusehen, da diese bei spontanen Atemzügen - also vom Lebewesen gewollten Atemzügen - nicht vorkommen und mit (erzwungenen) Auslösungen durch das Beatmungsgerät korrelieren können. Es ist also streng zwischen kurzen Auslöseverzögerungen und Auslöseverzögerungen (allgemein) zu unterscheiden. In bevorzugten Ausführungsformen des Systems werden Auslöseverzögerungen für die Auslösung einer Inspiration durch das Beatmungsgerät ohne Atemanstrengung des Lebewesens nicht bestimmt.

Ferner wird darauf hingewiesen, dass die verwendeten Einheiten, insbesondere Zeiteinheiten, in allen bekannten Schreibweisen gleichbedeutend sind. So entspricht die Einheit "s" ebenso wie die Einheit "sec" Sekunden und kann in den drei Formen (s, sec, Sekunde(n)) austauschbar verwendet werden. Gleiches gilt auch für Minuten, welche mitunter

als "min" abgekürzt werden. Insbesondere bei der Verwendung von "min" wird darauf hingewiesen, dass die Einheit "min" nicht mit der Minimum-Funktion min() zu verwechseln ist. Insbesondere ist an Stellen, an denen die Funktion min() gemeint ist, diese in der Ausführung mit zumindest den Klammern gesetzt, wobei in der Klammer entsprechend die Attribute/Werte/Aktionen aufgeführt sind.

Das Beatmungsgerät ist zumindest mit einer Sensoreinheit, einer Aufbereitungseinheit, einer Berechnungseinheit, einer Erkennungseinheit, einer Steuerungseinheit sowie einer Atemgasquelle wie zum Beispiel einer Gebläse-/Ventileinheit ausgestattet. Die Sensoreinheit, die Aufbereitungseinheit, die Berechnungseinheit, die Erkennungseinheit und die Steuerungseinheit verfügen beispielsweise jeweils einzeln oder auch gemeinsam über einen Prozessor, um die nachfolgenden Schritte, wie Berechnungen, Analysen und/oder Algorithmen, ausführen zu können.

Die Sensoreinheit ist ausgebildet, um Messwerte wie Druck und Fluss mit Bezug auf das Lebewesen sowie das Beatmungsgerät - etwa den durch die Atemgasquelle bereitgestellten Gasfluss und -druck - zu messen. In manchen Ausführungsformen kann vorgesehen sein, dass die Sensoreinheit auch ausgebildet ist, um weitere Messwerte wie Temperatur, Feuchtigkeit, Gaskonzentrationen, Volumina etc. zu messen.

Die Aufbereitungseinheit ist beispielsweise dazu eingerichtet, die Messwerte der Sensoreinheit aufzubereiten und dem System zur Verfügung zu stellen.

Die Berechnungseinheit ist dazu eingerichtet, beispielsweise mittels eines Prozessors die aufbereiteten Messwerte sowie weitere Daten, Werte und Informationen weiterzuverarbeiten. Insbesondere ist die Berechnungseinheit dazu eingerichtet, die folgenden Berechnungsschritte durchzuführen.

Die Erkennungseinheit ist unter anderem dazu eingerichtet, anhand der berechneten Daten, Werte und Informationen verschiedene Zustände (Inspirationsphase, Exspirationsphase etc.) der Atmung des Lebewesens zu bestimmen. Insbesondere ist die Erkennungseinheit dazu eingerichtet, Asynchronien zwischen Beatmungsgerät und Lebewesen in Form von kurzen Auslöseverzögerungen und verpassten Atemzügen sowie in manchen Ausführungsformen auch in Form von falschen Auslösungen zu erkennen.

Die Steuerungseinheit ist ausgebildet, um zumindest anhand der durch die Erkennungseinheit erkannten Asynchronien das Beatmungsgerät zumindest teilweise und zumindest zeitweise automatisch zu steuern.

Die Erkennung von Asynchronien in Form von verpassten Atemzügen und kurzen Auslöseverzögerungen sowie, in manchen Ausführungsformen, von falschen Auslösungen basiert in dem System auf den effektiven Lungenparametern des Lebewesens, wie zum Beispiel dem effektiven Atemwegswiderstand R und der effektiven Lungenelastizität E. In der Regel werden Lungenparameter bei Lebewesen unter Narkose und komplett passiver Atmung bestimmt. Da hier die Lungenparameter aber während der Beatmung und einer aktiven Atmung des Lebewesens gefittet bzw. ermittelt werden, werden die Parameter als "effektive" Parameter bezeichnet.

Diese effektiven Parameter können beispielsweise über ein mathematisches Lungenmodell, etwa das Ein-Kompartment-Lungenmodell, bestimmt werden. In manchen Ausführungsformen kann beispielsweise auch ein anderes mathematisches Lungenmodell, etwa das Zwei-Kompartment-Lungenmodell oder ein nichtlineares Lungenmodell, genutzt werden.

Das Ein-Kompartment-Lungenmodell beschreibt über

$$EV + R\dot{V} = P - P_b$$

den Zusammenhang zwischen der effektiven Lungenelastizität E, dem effektiven Atemwegswiderstand R, dem Tidalvolumen V, dem Atemfluss V, einem Basisdruck $P_b$ (beispielsweise dem positiven endexspiratorischen Druck, kurz PEEP) und dem durch das Beatmungsgerät bereitgestellten Druck P.

Die effektive Lungenelastizität E und der effektive Atemwegswiderstand R können beispielsweise über eine multiple lineare Regression aus dem Ein-Kompartment-Lungenmodell bestimmt werden. Dazu wird angenommen, dass der Druck P, der Basisdruck $P_b$, das Tidalvolumen V und der Atemfluss V für jeden individuellen Atemzug bekannt sind. Dazu wird

$$A = \begin{pmatrix} V_0 & \dot{V}_0 \\ V_1 & \dot{V}_1 \\ V_2 & \dot{V}_2 \\ \vdots & \vdots \\ V_n & \dot{V}_n \end{pmatrix},$$

definiert, wobei die Indizes Messpunkte zu verschiedenen Zeitpunkten bezeichnen. Weiter werden

$$x = \begin{pmatrix} E \\ R \end{pmatrix}$$

und

$$Rhs = \begin{pmatrix} P_0 - P_b \\ P_1 - P_b \\ P_2 - P_b \\ \vdots \\ P_n - P_b \end{pmatrix},$$

definiert, sodass das Ein-Kompartment-Lungenmodell als

$$Ax = Rhs$$

ausgedrückt werden kann.

Um die effektive Lungenelastizität E und den effektiven Atemwegswiderstand R zu bestimmen, wird die Technik der multiplen linearen Regression angewandt, welche ein lineares Gleichungssystem der Form

$$A^t Ax = A^t Rhs,$$

ergibt. $A^t$ stellt dabei die transponierte Matrix von A dar. Daraus ergibt sich also

$$\begin{pmatrix} \sum_i V_i^2 & \sum_i V_i \dot{V}_i \\ \sum_i V_i \dot{V}_i & \sum_i \dot{V}_i^2 \end{pmatrix} \begin{pmatrix} E \\ R \end{pmatrix} = \begin{pmatrix} \sum_i V_i (P_i - P_b) \\ \sum_i \dot{V}_i (P_i - P_b) \end{pmatrix}.$$

Dieses lineare Gleichungssystem kann für E und R gelöst werden und ergibt eine Näherung des effektiven Atemwegwiderstandes R und der effektiven Lungenelastizität E für einen konkreten Atemzug. Das gemittelte Quadrat des Residuums

$$r = \sqrt{\frac{1}{n}[(P - P_b) - Ax]^2}$$

kann genutzt werden, um eine Abschätzung über die Genauigkeit der Näherung von E und R für einen konkreten Atemzug zu bestimmen.

Die Lungenparameter E und R können je nach verschiedenen Einflüssen, wie z. B. Position des Lebewesens, Schlafphase etc., mit der Zeit variieren. Auch können die bestimmten Werte für E und R für unterschiedliche Atemzüge eine unterschiedliche Genauigkeit aufweisen. Um die zeitlichen Veränderungen und die variierende Genauigkeit mit einzubeziehen, werden die bestimmten Lungenparameter vor der Erkennung der Asynchronien gefiltert. Dazu können über verschiedene Methoden die Mittelwerte bzw. Durchschnitte, beispielsweise gewichtete Mittelwerte, bestimmt werden.

Die exponentiell gewichteten Mittelwerte werden über

$$\bar{E}_{n+1} = \lambda \bar{E}_n + (1 - \lambda) E_{n+1}$$

und

$$\bar{R}_{n+1} = \lambda \bar{R}_n + (1 - \lambda) R_{n+1}$$

bestimmt, wobei die Indizes die Nummer des Atemzuges bezeichnen und $\lambda$ der sogenannte Vergessensfaktor ist, welcher genutzt wird, um die vorherigen sowie den aktuellen Atemzug in dem Mittelwert entsprechend zu gewichten. Der

Vergessensfaktor λ wird dabei zum Beispiel entsprechend der Zeitkonstante

$$\tau = -\frac{\Delta t}{\ln(\lambda)}$$

gewählt. Die Zeitkonstante τ entspricht beispielsweise etwa 110 sec bis 180 sec, entsprechend einem 2-Minuten-Intervall, wobei eine typische Atemzugsdauer $\Delta t$ im Bereich von 3,2 sec bis 4,0 sec angenommen wird. Eine typische Atemzuglänge kann generell in einem Bereich von 2,0 sec bis 6,0 sec angenommen werden. Bevorzugt kann beispielsweise eine Atemzuglänge zwischen 3,0 sec und 4,0 sec angenommen werden. In manchen Ausführungsformen kann statt des 2-Minuten-Intervalls auch eine andere Intervalllänge gewählt werden. Die Länge des Zeitintervalls kann beispielsweise auch zwischen 1 Minute und 10 Minuten gewählt werden. Entsprechend kann in manchen Ausführungsformen auch der Vergessensfaktor λ angepasst werden, typischerweise mit einem Wert von 0,01 bis 1,00. Nimmt man beispielsweise eine Atemzuglänge zwischen 3,0 sec und 4,0 sec an und betrachtet man ein Intervall mit einer Länge zwischen 100 sec und 180 sec, so kann λ zwischen 0,90 und 1,00, bevorzugt zwischen 0,95 und 0,99 liegen. Beispielsweise wird der Vergessensfaktor λ an die angenommene typische Atemzugsdauer und das betrachtete Zeitintervall angepasst. Die Atemzugsdauer kann für die Berechnung ebenfalls variabel gewählt werden und beispielsweise in einem Bereich von 1,0 sec bis 15,0 sec, bevorzugt zwischen 2,0 sec und 8,0 sec, liegen.

In manchen Ausführungsformen können die Mittelwerte von R und E auch durch andere Methoden bestimmt werden, beispielsweise durch einen linear gewichteten Durchschnitt und/oder einen logarithmischen Mittelwert und/oder einen quadratischen und/oder kubischen Mittelwert und/oder ein Gastwirth-Cohen-Mittel und/oder eine Kombination verschiedener Mittelwerte bzw. Methoden zur Bestimmung von (gewichteten) Mittelwerten.

Die Berechnung der effektiven Lungenelastizität E und des effektiven Atemwegswiderstandes R bildet die Grundlage zur Bestimmung weiterer Parameter wie des Atemanstrengungsflusses $V_{eff}$ und des erwarteten Atemflusses $\dot{V}_{exp}$, über welche wiederum Asynchronien wie verpasste Atemzüge, die Auslöseverzögerung und gegebenenfalls falsche Auslösungen durch das Beatmungsgerät erkannt werden.

Die Analyse des Atemflusses ist ein grundlegendes Werkzeug zur Bestimmung von Asynchronien zwischen dem Patienten und dem Beatmungsgerät. Um Anomalien im Atemfluss zu erkennen, ist es von Vorteil zu wissen, wie das Verhalten des Atemflusses zu erwarten ist. Unterschiede zwischen dem bestimmten und dem erwarteten Atemfluss können zum Beispiel auf Asynchronien hinweisen. Für beatmete Patienten ist der Atemfluss stark von dem Druck abhängig, welcher durch das Beatmungsgerät erzeugt wird. Löst man die Differentialgleichung des Ein-Kompartment-Lungenmodells

$$P - P_b = \bar{E}V_{exp} + \bar{R}\dot{V}_{exp}.$$

durch Einspeisen des Drucks P, welcher vom Beatmungsgerät erzeugt wird, und der vorher gemittelten bestimmten Lungenparameter $\bar{E}$ und $\bar{R}$, kann der erwartete Fluss $\dot{V}_{exp}$ bestimmt werden. Es wird zudem angenommen, dass das erwartete Volumen $V_{exp}$ zu Beginn eines Atemzuges null ist. Der bestimmte Atemfluss stellt dabei den Atemfluss des Patienten dar, welcher aus dem vom Beatmungsgerät gemessenen Fluss unter Abzug der Leckage (vermutet, geschätzt oder gemessen) und anderen Ungenauigkeiten oder Einflüssen berechnet wird.

Wenn der bestimmte Atemfluss und der erwartete Atemfluss $\dot{V}_{exp}$ bekannt sind, kann die Abweichung, das Residuum, als Atemanstrengungsfluss $\dot{V}_{eff}$ mit

$$\dot{V}_{eff} = \dot{V} - \dot{V}_{exp}$$

bestimmt werden, welcher Informationen bezüglich der (unerwarteten) Atemanstrengungen des Lebewesens enthält. Der Atemanstrengungsfluss $\dot{V}_{eff}$ und/oder der zeitliche Verlauf des Atemanstrengungsflusses $\dot{V}_{eff}$ beinhaltet Schlüsselinformationen für den Algorithmus zur Detektion der Asynchronien.

Das System ist eingerichtet, um den Algorithmus zur Erkennung von Asynchronien basierend auf der Analyse des Atemanstrengungsflusses und weiteren Signalen, Daten oder Werten auszuführen.

Als ein verpasster Atemzug wird eine Atemanstrengung des Patienten definiert, welche nicht von dem Beatmungsgerät erkannt wird und daher nicht vom Beatmungsgerät durch Erhöhen des Drucks und/oder Flusses unterstützt wird.

Der respiratorische (oder auch bestimmte) Atemfluss ist bei einem verpassten Atemzug typischerweise durch ein kleines positives Maximum, welches von einem kleinen negativen Minimum gefolgt wird, charakterisiert.

Die Erkennung der verpassten Atemzüge wird zum Beispiel durch einen Machine-Learning-Algorithmus ausgeführt. Dazu werden initial reale Daten von Lebewesen hinsichtlich der verpassten Atemzüge manuell ausgewertet und

dem Machine-Learning-Algorithmus zur Verfügung gestellt. Der Machine-Learning-Algorithmus kann dann auf unbekannte Daten oder während der Anwendung des Beatmungsgerätes angewendet werden. Beispielsweise wird der Machine-Learning-Algorithmus nach Anlernen durch die manuell ausgewerteten Daten auf dem Beatmungsgerät gespeichert und durch die Erkennungseinheit ausgeführt.

Es ist zudem denkbar, dass der Machine-Learning-Algorithmus auf dem Beatmungsgerät weitere Lernfortschritte erzielt und diese von Zeit zu Zeit über eine Schnittstelle, beispielsweise (anonymisiert) an einen Server oder eine Cloud, gesendet werden. Über diesen Server können die gesammelten Daten genutzt werden, um den Algorithmus zu verbessern, beispielsweise durch eine höhere Genauigkeit in der Erkennung. So kann es sein, dass das Beatmungsgerät den Machine-Learning-Algorithmus von Zeit zu Zeit mit Daten von dem Server aktualisiert bzw. weitere Lerndaten zur Verfügung stellt. Auch ist es denkbar, dass der Machine-Learning-Algorithmus von Zeit zu Zeit außerhalb des Beatmungsgerätes weiterlernt, beispielsweise durch weitere manuell ausgewertete Daten, und die Lernfortschritte von Zeit zu Zeit an das Beatmungsgerät übertragen werden, sodass der Machine-Learning-Algorithmus weiter verbessert wird.

Die Merkmale, anhand derer der Machine-Learning-Algorithmus die verpassten Atemzüge anhand des Atemanstrengungsflusses erkennt, sind zum Beispiel:

1. das lokale Maximum des bestimmten Atemflusses muss zwischen zwei Minima des Atemanstrengungsflusses liegen;
2. Differenz zwischen der zeitlichen Lage des lokalen Maximums des Atemanstrengungsflusses und dem entsprechenden linken Minimum;
3. Differenz zwischen der zeitlichen Lage des lokalen Maximums des Atemanstrengungsflusses und dem entsprechenden rechten Minimum;
4. Differenz zwischen den Werten des lokalen Maximums des Atemanstrengungsflusses und dem entsprechenden linken Minimum;
5. Differenz zwischen den Werten des lokalen Maximums des Atemanstrengungsflusses und dem entsprechenden rechten Minimum;
6. erwarteter Atemfluss zum Zeitpunkt des lokalen Maximums des Atemanstrengungsflusses;
7. Zeit zwischen dem Maximum des Atemanstrengungsflusses und dem erwarteten Auslösezeitpunkt.

Wird zumindest eines dieser Merkmale erkannt, kann der Algorithmus auf einen verpassten Atemzug schließen. Bevorzugt ist vorgesehen, dass mehrere, beispielsweise zwei oder vier oder alle der genannten Merkmale zutreffen müssen, damit der Algorithmus auf einen verpassten Atemzug schließt.

Der erwartete Auslösezeitpunkt $\bar{t}$ wird durch die Länge des letzten, durch das Lebewesen selbst ausgelösten, spontanen Atemzugs $t_{current}$ und der letzten erwarteten Auslösezeit $\bar{t}_{old}$

$$\bar{t} = (1-\gamma)\bar{t}_{old} + \gamma t_{current},$$

bestimmt, wobei $\gamma$ ein Faktor zur Gewichtung von $\bar{t}_{old}$ und $t_{current}$ ist. Der Wert von $\gamma$ kann dabei zwischen 0,01 und 1,00 gewählt werden, bevorzugt zwischen 0,1 und 0,5. Beispielsweise kann ein Wert für $\gamma$ von 1/4 und/oder 1/3 und/oder 1/2 als sinnvoll erachtet werden. Der erwartete Auslösezeitpunkt kann in manchen Ausführungsformen beispielsweise auch durch einen fixen Wert festgesetzt werden. In manchen Ausführungsformen wird der Auslösezeitpunkt durch einen gleitenden Durchschnitt, beispielsweise über ein Intervall, welches einem Zeitraum zwischen 3 und 100 Atemzügen entspricht, bestimmt. Auch andere Methoden zur Bestimmung eines Durchschnitts oder Mittelwerts können hier genutzt werden.

Das Beatmungsgerät ist zudem eingerichtet, um Asynchronien anhand von Auslöseverzögerungen oder kurzen Auslöseverzögerungen zu erkennen.

Auslöseverzögerungen treten zu Beginn der spontanen Atmung oder der Atemanstrengung auf und sind als der Zeitabstand zwischen der Aktivierung der respiratorischen Muskeln des Patienten und der Auslösung der Atmungsunterstützung durch das Beatmungsgerät definiert. Typischerweise sind die Auslöseverzögerungen durch ein lokales Maximum des Atemanstrengungsflusses charakterisiert. Alternativ oder ergänzend sind die Auslöseverzögerungen durch den Abstand des Maximums in der Atemanstrengung vom Auslösezeitpunkt des Beatmungsgerätes charakterisiert.

Eine Auslöseverzögerung wird für alle ausgelösten Atemzüge bestimmt. Ist die Auslöseverzögerung kleiner als ein oder gleich einem Schwellenwert (z. B. 0,1 s), welcher in der Regel zu kurz für einen spontanen Atemzug ist, wird diese Auslöseverzögerung als "kurze Auslöseverzögerung" bezeichnet. Während die Auslöseverzögerung für alle Atemzüge (spontan und durch das Beatmungsgerät vorgegeben) bestimmt wird, findet eine Analyse auf kurze Auslöseverzögerungen nur für spontane, also nicht durch das Beatmungsgerät vorgegebene Atemzüge statt. Die Bestimmung der Auslöseverzögerung erfolgt beispielsweise folgendermaßen:

Zunächst wird der Atemanstrengungsfluss $\dot{V}_{eff}$ durch einen Tiefpass-Filter, beispielsweise ein Butterworth-Filter (z. B. erster, zweiter, dritter, vierter und/oder fünfter Ordnung) und/oder ein Legendre-Filter und/oder ein Tschebyscheff-Filter und/oder ein Bessel-Filter und/oder ein Cauer-Filter und/oder ein Gauß-Filter und/oder ein Raised-Cosine-Filter und/oder ein TBT-Filter, mit einer Grenzfrequenz von 3 Hz gefiltert, um $\dot{V}_{eff}^{lowpass}$ zu erhalten.

Daraufhin wird die erwartete Amplitude A für den aktuellen Atemzug i berechnet mit

$$A = 2\ mean\left[\dot{V}_{exp}(P_{set} = IPAP\ \&\ t_i \le t < t_{i+1})\right],$$

wobei $P_{set}$ den Sollwert des Drucks (pressure setpoint), $t_i$ den Startzeitpunkt des Atemzuges i und IPAP den inspiratorischen positiven Atemwegsdruck (IPAP) darstellt.

Unmittelbar vor dem Auslösepunkt der Inspiration beginnend und in der Exspirationsphase rückwärtsgehend, wird jeder Messpunkt k geprüft, ob er Teil der Auslöseverzögerung ist. Zunächst wird dazu die Anzahl $n_{highFlow}$ der Messpunkte k mit vergleichsweise hohen Atemflüssen über einem Schwellenwert von

$$\dot{V}(k) \ge a_1 A,$$

mit $a_1$ zwischen 0,025 und 0,075, beispielsweise zwischen 0,050 und 0,060, berechnet.

Darauf wird mit dem letzten Messpunkt, welcher über dem Schwellenwert lag, erneut begonnen und weiter zurückgegangen, wobei die Anzahl $n_{increasingFlow}$ der Messpunkte k gezählt wird, welche dadurch charakterisiert werden, dass sie einen anwachsenden

Atemanstrengungsfluss aufweisen und jeder Messpunkt k folgende Bedingungen erfüllt:

$$\dot{V}_{eff}^{lowpass}(k) > 0$$

*und*

$$\{\{\frac{d}{dt}\dot{V}_{eff}^{lowpass}(k) > 0$$

*und*

$$\frac{d}{dt}\dot{V}_{eff}^{lowpass}(k) > a_2 \max\left(\frac{d}{dt}\dot{V}_{eff}^{lowpass}\left(k{:}\,k + n_{increasingFlow}\right)\right)\}$$

*oder*

$$\max\left(\frac{d^2}{dt^2}\dot{V}_{eff}^{lowpass}\left(k{:}\,k + n_{increasingFlow}\right)\right) < 0\},$$

wobei a2 zwischen 0,3 und 0,6, beispielsweise als 0,4 oder 0,5, gewählt wird. Die Gesamtzahl der Testpunkte, welche möglichen Auslöseverzögerungen entsprechen, ist

$$n = n_{increasingFlow} + n_{highFlow}.$$

Abschließend wird geprüft, ob die mögliche Auslöseverzögerung gültig ist. Dies ist beispielsweise der Fall, falls

$$mean\left(\frac{d}{dt}\dot{V}(k_{start}{:}\,k + n - 1)\right) > a_3 A$$

*und*

$$\dot{V}(k_{start} + n - 1) - \dot{V}(k_{start}) > a_1 A$$

*und*

$$\dot{V}_{eff}^{lowpass}\left(k_{start} + n_{increasingFlow}\right) - \dot{V}_{eff}^{lowpass}(k_{start}) > 0,$$

mit $a_3$ zwischen 0,05/s und 0,50/s, bevorzugt zwischen 0,20/s und 0,30/s, und $k_{start}$ als erstem Messpunkt der möglichen Auslöseverzögerung. Ist eine mögliche Auslöseverzögerung nicht gültig, wird n auf null gesetzt. Die Auslöseverzögerung ergibt dann

$$t_{triggerDelay} = n\ 0{,}01s.$$

Eine ungültige Auslöseverzögerung wird bei spontanen Atemzügen also entsprechend einer kurzen Auslöseverzögerung gewertet. Wie vorangehend dargelegt wird vom Beatmungsgerät eine Auslöseverzögerung $t_{triggerDelay}$ unterhalb eines bestimmten Schwellenwerts oder gleich einem bestimmten Schwellenwert als kurze Auslöseverzögerung gewertet. Für den Schwellenwert kann beispielsweise ein Wert zwischen 0,01 sec und 0,5 sec, bevorzugt zwischen 0,05 sec und 0,15 sec, eingestellt werden.

In manchen Ausführungsformen ist das Beatmungsgerät ferner dazu eingerichtet, falsche Auslösungen anhand folgender Auswertungen oder Berechnungen zu erkennen.

Unter falschen Auslösungen sind Auslösungen zu verstehen, welche durch das Beatmungsgerät erkannt wurden, aber nicht durch den Patienten angefordert wurden, bei denen der Patient also keine Atemanstrengung gezeigt hat. Diese können durch Analyse der Daten im Zeitraum um den Auslösezeitpunkt $t_{trig}$ des Atemgerätes erkannt werden. Der normalisierte Atemfluss vor der Auslösung

$$f_{pre} = mean\left(\dot{V}\left(t_{trig} - t_{pre} \leq t < t_{trig}\right)\right)/A$$

wird in einem Zeitraum berechnet, welche vor $t_{trig}$ liegt, wobei mit $t_{pre}$ zwischen 0,08 sec und 0,2 sec gerechnet wird. Der normalisierte Atemfluss nach der Auslösung

$$f_{post} = \frac{mean\left(\dot{V}\left(t_{trig} \leq t \leq t_{trig} + t_{post}\right)\right)}{mean\left(\dot{V}_{exp}\left(t_{trig} \leq t \leq t_{trig} + t_{post}\right)\right)}$$

wird aus dem Zeitraum nach $t_{trig}$ berechnet, wobei beispielsweise mit einem Wert für $t_{post}$ zwischen 0,1 s und 0,5 s gerechnet werden kann. Eine falsche Auslösung des Beatmungsgerätes wird beispielsweise erkannt, wenn $f_{pre}$ kleiner als ein Wert zwischen 0,01 und 0,10, beispielsweise 0,05, ist und $f_{post}$ kleiner als ein Wert zwischen 0,5 und 0,9, beispielsweise 0,7, ist.

Das System ist beispielsweise auch ausgebildet, um die erkannten kurzen Auslöseverzögerungen sowie die verpassten Atemzüge heranzuziehen, um die Auslöseempfindlichkeit des Beatmungsgerätes automatisch einzustellen. Die automatische Auslöseeinstellung passt die Empfindlichkeit des Beatmungsgerätes zum Auslösen für das Einatmen automatisch an, um die individuellen Bedürfnisse des Patienten zu erfüllen.

In der Regel findet die Einstellung der Auslöseempfindlichkeit über eine Angabe von Stufen statt. Die Stufen der Auslöseempfindlichkeit können in manchen Ausführungsformen anhand von Atemflusswerten, beispielsweise in l/min, wiedergegeben werden. So sind beispielsweise Abstufungen von 1 l/min bis 15 l/min in Schritten von 1 l/min denkbar. Darüber hinaus können auch Zwischenstufen eingeführt werden, etwa in 0,5-l/min-Schritten. Auch andere Bezeichnungen für die Stufen der Auslöseempfindlichkeit, beispielsweise mit Zahlen, Buchstaben, Beschreibungen und/oder Symbolen, sind möglich. Beschreibungen könnten zum Beispiel die Form "sehr empfindlich", "empfindlich", "wenig empfindlich", "nicht empfindlich" annehmen. Neben der Bezeichnung der Stufen (numerisch, alphabetisch, symbolisch, durch Flusswerte etc.) kann auch die Anzahl und/oder der Abstand der verfügbaren Stufen variieren.

In manchen Ausführungsformen des Systems kann die Auslöseempfindlichkeit beispielsweise manuell in Stufen zwischen 1 und 8 eingestellt werden, wobei beispielsweise kleinere Werte einer höheren Empfindlichkeit entsprechen - beispielsweise zusammenhängend mit einem geringeren Atemfluss, welcher die Unterstützung durch das Beatmungsgerät auslöst. Für die automatische Auslösungseinstellung wird beispielweise eine weitere Stufe 0 der Auslöseempfindlichkeit hinzugefügt, welche einer noch höheren Empfindlichkeit entspricht. Diese höhere Auslöseempfindlichkeit kann

besonders für COPD-Patienten mit einem intrinsischen positiven endexspiratorischen Druck (iPEEP) nützlich sein, aber auch für andere Krankheitsbilder oder Fälle, in denen das System oder Beatmungsgerät eingesetzt wird. Die automatische Auslöseeinstellung ist so ausgelegt, dass dadurch die Empfindlichkeitsstufen zwischen 0 und 3 eingestellt werden. In manchen Ausführungsformen kann durch die automatische Auslöseeinstellung auch eine Auslöseempfindlichkeit zwischen 0 und 8 eingestellt werden. Eine zu hohe Empfindlichkeit (entspricht kleinen Stufen der Auslöseempfindlichkeit) kann unter Umständen bei Einsatzgebieten außerhalb von Beatmungstherapien als nicht erwünscht oder störend angesehen werden. Die Stufen geben unter anderem Schwellenwerte für den Atemfluss wieder, bei denen eine Inspiration bzw. die Unterstützung bei der Inspiration ausgelöst wird.

Die Anpassung der Auslöseempfindlichkeit über die automatische Einstellung (auch Auto-Trigger-Funktion) beruht auf der Anzahl der festgestellten verpassten Atemzüge und kurzen Auslöseverzögerungen in einem vorbestimmten Zeitintervall, z. B. zwei Minuten. Hier können auch andere Zeitintervalle genutzt werden, welche idealerweise an die Zeitintervalle der Berechnung oder Erkennung der Asynchronien angepasst sind. Die folgenden, sich gegenseitig ausschließenden Regeln gelten für die Anpassung der Auslöseempfindlichkeit

$$1.\ IF\ (\#ShoTrigDel \geq b\ \#IneffEff)\ \&\ (\#ShoTrigDel \geq c) \rightarrow trigSens = \min(trigSens + 1{,}3)$$

$$2.\ IF\ [(\#IneffEff \geq b\ \#ShoTrigDel)\ \&\ (\#IneffEff \geq c)] \rightarrow trigSens = \max(trigSens - 1{,}0)$$

$$3.\ IF\ (\#ShoTrigDel \leq d)\ \&\ (\#ShoTrigDel_{old} \leq d)\ \&\ (trigSens = trigSens_{old}) \rightarrow trigSens = \max(trigSens - 1{,}1)$$

wobei #IneffEff die Anzahl der registrierten, verpassten Atemzüge in dem Zeitintervall angeben, #ShoTrigDel für die Anzahl der kurzen Atemauslöseverzögerungen im gleichen Intervall steht, der Index "old" das vorhergehende Zeitintervall beschreibt, die Parameter b, c, d beispielsweise in einem Bereich

*b* = 0 bis 6,

*c* = 0 bis 6,

*d* = 0 bis 6

sind und trigSens das aktuelle Auslöseempfindlichkeitslevel ist. Insbesondere können die Parameter b, c, d Werte von 0 bis 6 annehmen, beispielsweise von 0 bis 4 oder von 0 bis 3. Es kann dabei vorgesehen sein, dass b = c und c > d gilt. Der Parameter d hat dabei beispielsweise einen Wert unter 1. Das Komma separiert in diesem Fall die zwei Parameter der Mimimum-Funktion min() und der Maximum-Funktion max(). Bei min(i,j) und max(i,j) handelt es sich um Funktionen. Dabei würde min() den kleineren Wert von i und j zurückliefern, wohingegen max() den größeren Wert als Resultat hätte. Beispiel: min(2,4) = 2 und max(2,4) = 4. Dementsprechend bedeutet trigSens = min(trigSens + 1,4), dass die Variable trigSens um 1 erhöht wird, aber maximal den Wert 4 annehmen kann. Beispiel: trigSens hat den Wert 3. Nach der Zuweisung trigSens = min(trigSens + 1,4), hat trigSens den neuen Wert min(4,4) = 4. Hat trigSens nun den Wert 4 und die Zuweisung trigSens = min(trigSens + 1,4) wird ausgeführt, so hat trigSens den neuen Wert min(5,4) = 4. TrigSens kann auf diese Art und Weise also nicht größer werden als 4.

Statt Zahlenwerte für TrigSens sind, wie zuvor beschrieben, auch andere Ausdrücke, beispielswiese Worte, Buchtstaben und/oder Symbole, möglich. Werden keine Zahlenwerte für TrigSens verwendet, muss der Anpassungsablauf entsprechend so angepasst werden, dass die jeweilige Stufenanpassung erfolgen kann. Es kann ergänzend oder alternativ vorgesehen sein, dass die Anzeige der Auslöseempfindlichkeit mit Buchstaben, Symbolen, Beschreibungen und/oder Flusswerten erfolgt, wobei der jeweils angezeigten Art eine entsprechende Zahlenskala zugeordnet ist.

In manchen Ausführungsformen können b, c, d auch andere Werte annehmen, beispielsweise zwischen 1 und 6, bevorzugt zwischen 2 und 4. Die hier angegebenen Wertebereiche für b, c, d können in manchen Ausführungsformen auch mit den oben genannten gemischt werden. Beispielsweise kann für b, c ein Wertebereich von 1 bis 6 zugrunde gelegt werden, während für d ein Wertebereich von 0 bis 6 gilt. Weiter ist es beispielsweise denkbar, dass durch das 2-Minuten-Intervall das gesamte vorhergehende Intervall beschrieben wird und das nächste Intervall beginnt, nachdem das vorherige Intervall beendet wurde. Das erste Intervall beginnt also beispielsweise bei Sekunde 0 und dauert bis zur

Sekunde 120 an. Das zweite Intervall schließt sich nahtlos an und dauert von der Sekunde 120 bis zur Sekunde 240. Die gewählte Intervalllänge kann darüber hinaus in einem Bereich zwischen 60 sec und 240 sec liegen, bevorzugt zwischen 100 sec und 180 sec.

Die Anzahl der verpassten Atemzüge und die Anzahl der kurzen Auslöseverzögerungen werden für Fälle mit hohen Leckageflüssen ungenauer. Daher wird, falls die durchschnittlichen Leckageflüsse in dem 2-Minuten-Intervall über einem Wert zwischen 15 l/min und 50 l/min, beispielsweise 25 l/min, liegen, die Auslöseempfindlichkeit als eine durchschnittliche Auslöseempfindlichkeit

$$\overline{T}_{new} = (1 - \lambda_{Leak})\overline{T}_{old} + \lambda_{Leak} T$$

eingestellt, welche sich aus den vorherigen Zeiträumen mit geringeren oder keinen Leckageflüssen berechnet. Dabei sind $\overline{T}_{new}$ und $\overline{T}_{old}$ die neuen (Index "new") und die alten (Index "old") Durchschnittswerte. $T$ ist die für den aktuellen Zeitraum berechnete Auslösesensitivität und $\lambda_{Leak}$ ist der dazugehörige Vergessensfaktor, welchem beispielsweise ein Wert zwischen 0,01 und 1,00 zugeordnet werden kann. Bevorzugt liegt der Wert zwischen 0,05 und 0,40 und/oder zwischen 0,15 und 0,30. Der Vergessensfaktor ist ein Faktor, welcher einberechnet wird, um den vorhergehenden und den aktuellen Zeitraum entsprechend dem 2-Minuten-Intervall zu gewichten, oder die Zeitskala der Filterung - hier ungefähr zwei Minuten - definiert. In manchen Ausführungsformen wird beispielsweise in Abhängigkeit vom Leckagefluss bestimmt, ob und/oder in welchem Maß Anpassungen der Auslöseempfindlichkeit $\overline{T}$ vorgenommen werden. Beispielsweise wird beim Überschreiten eines bestimmten Leckageflusses die Anpassung der Auslöseempfindlichkeit $\overline{T}$ ausgesetzt. Der Schwellenwert der Leckageflüsse, über dem die Anpassung ausgesetzt wird, liegt beispielsweise im Bereich zwischen 15 l/min und 50 l/min, bevorzugt zwischen 20 l/min und 30 l/min. Der Schwellenwert kann sich beispielsweise auch auf einen durchschnittlichen Wert der Leckageflüsse beziehen. So kann eine Aussetzung der Anpassung der Auslöseempfindlichkeit $\overline{T}$ auch ab und/oder über einem Schwellenwert von durchschnittlichen Leckageflüssen zwischen 15 l/min und 50 l/min, bevorzugt zwischen 20 l/min und 30 l/min, erfolgen. Die Auslöseempfindlichkeit $\overline{T}$ wird beispielsweise nicht für Phasen mit hohen durchschnittlichen Leckageflüssen über 25 l/min angepasst.

In manchen Ausführungsformen ist das System auch dazu eingerichtet, verfrühte Exspirationstrigger, sogenannte "early cyclings", zu erkennen und als Asynchronie zu werten. Verfrühte Exspirationstrigger treten auf, wenn das Beatmungsgerät von der Inspiration in die Exspiration umschaltet, obwohl der Anwender oder Patient die Inspirationsphase noch nicht abgeschlossen hat. Durch den Druckabfall wird der Patient dazu gebracht, auszuatmen, wonach typischerweise ein kurzer Anstieg des Atemflusses folgt, da der Patient seine Inspiration noch nicht abgeschlossen hat bzw. weiter einatmen möchte. Häufig passt sich der Patient letztlich dem Beatmungsgerät an und beginnt mit der Exspiration.

Bei der Bestimmung der verfrühten Exspirationstrigger sind vier grundsätzliche Punkte im Atemflussverlauf zu beachten: der Start der Druckrampe von Inspiration zu Exspiration (durch das Beatmungsgerät), der Endpunkt der Druckrampe, die Position des lokalen Maximums des Atemflusses, welches nicht weiter als eine bestimmte Zeitspanne nach dem Endpunkt der Druckrampe lokalisiert sein sollte, sowie des lokalen Minimums, welches innerhalb einer bestimmten Zeitspanne nach dem Endpunkt der Druckrampe folgen sollte. Die Zeitspanne für das lokale Maximum nach dem Endpunkt der Druckrampe beträgt beispielsweise zwischen 0,1 Sekunden und 2 Sekunden, beispielsweise zwischen 0,3 und 0,4 Sekunden. Die Zeitspanne für das lokale Minimum nach dem Endpunkt der Druckrampe beträgt beispielsweise zwischen 0,2 Sekunden und 3 Sekunden, beispielsweise zwischen 0,5 Sekunden und 0,7 Sekunden. Die verfrühten Exspirationstrigger sind zu Beginn der Exspirationsphasen zu beobachten und werden durch ein lokales Maximum des bestimmten Atemflusses charakterisiert, welches beim erwarteten Atemfluss nicht zu beobachten ist. Entsprechend gibt der Atemanstrengungsfluss ein lokales Maximum nach der Zeit des verfrühten Exspirationstriggers wieder. Anstatt einer Druckrampe kann alternativ oder ergänzend eine Flussrampe verwendet oder betrachtet werden.

Ein verfrühter Exspirationstrigger wird beispielsweise detektiert, wenn zumindest eine der die folgenden Bedingungen zutrifft:

1. bestimmter Atemfluss (Beginn der Druckrampe) > 0;
2. bestimmter Atemfluss (Ende der Druckrampe) < 0;
3. 

   e * bestimmter Atemfluss (lokales Maximum) / bestimmter Atemfluss (Ende der Druckrampe) < erwarteter Atemfluss (Maximum) / erwarteter Atemfluss (Ende der Druckrampe);

4. 

   f * bestimmter Atemfluss (lokales Maximum) > bestimmter Atemfluss (Ende der Druckrampe);

5.

g * bestimmter Atemfluss (lokales Minimum) < bestimmter Atemfluss (Ende der Druckrampe) + bestimmter Atemfluss (lokales Maximum);

6.

(h < bestimmter Atemfluss (lokales Maximum) - bestimmter Atemfluss (Ende der Druckrampe)) ODER (h < bestimmter Atemfluss (lokales Maximum) - bestimmter Atemfluss (lokales Minimum));

7. bestimmter Atemfluss (lokales Minimum) < 0.

Dabei liegt der Faktor e in einem Bereich von 0,5 bis 2,0, bevorzugt zwischen 1 und 1,8, der Faktor f in einem Bereich von 0,9 bis 2,9, bevorzugt zwischen 1,7 und 2,3, der Faktor g in einem Bereich von 1 bis 3, bevorzugt zwischen 1,8 und 2,2, und h in einem Bereich von 2 l/min bis 10 l/min, bevorzugt zwischen 3 l/min und 7 l/min.

Es kann in manchen Ausführungsformen zur Erkennung verfrühter Exspirationstrigger auch vorgesehen sein, dass statt der Bedingungen 1 und 2 allgemein gelten sollte, dass der bestimmte Atemfluss zu Beginn der Druckrampe größer ist als der bestimmte Atemfluss zum Ende der Druckrampe. In manchen Ausführungsformen können die Schwellenwerte für die Bedingungen 1, 2 und 7 auch unabhängig voneinander über und/oder unter 0 (l/min) liegen.

In manchen Ausführungsformen ist vorgesehen, dass zur Erkennung verfrühter Exspirationstrigger mindestens 2 oder 4 oder mehr oder alle der genannten Bedingungen zutreffen müssen.

Es wird darauf hingewiesen, dass eine gemeinsame Erkennung der Asynchronien aus den Atemflüssen oder dem Atemanstrengungsfluss möglich ist. Die beschriebenen Möglichkeiten der Erkennung, welche beispielhaft einzeln beschrieben sind, können entsprechend kombiniert werden. Auch die folgenden Beschreibungen anhand der Figuren stellen zum Teil jeweils die Erkennung von einzelnen Asynchronien dar. Eine Kombination dieser Erkennungen, um beispielsweise gleichzeitig verpasste Atemzüge und/oder kurze Auslöseverzögerungen und/oder falsche Auslösungen und/oder verfrühte Exspirationstrigger zu erkennen, kann ebenfalls möglich sein. Entsprechend kann auch eine Steuerung der Auslöseempfindlichkeit zumindest teilweise auf Basis der gemeinsam erkannten Asynchronien erfolgen.

In manchen Ausführungsformen umfasst die Auslöseempfindlichkeit zumindest einen Wert für die Umschaltung des Beatmungsgerätes in die Inspirationsphase, bei dem die Einatmung des Anwenders unterstützt wird, sowie optional zumindest einen Wert für die Umschaltung in eine Exspirationsphase, welche den Anwender bei der Ausatmung unterstützt. Die Exspirationsphase unterscheidet sich beispielsweise von der Inspirationsphase dadurch, dass ein niedrigerer Druck und/oder geringerer Fluss durch das Beatmungsgerät vorgegeben wird.

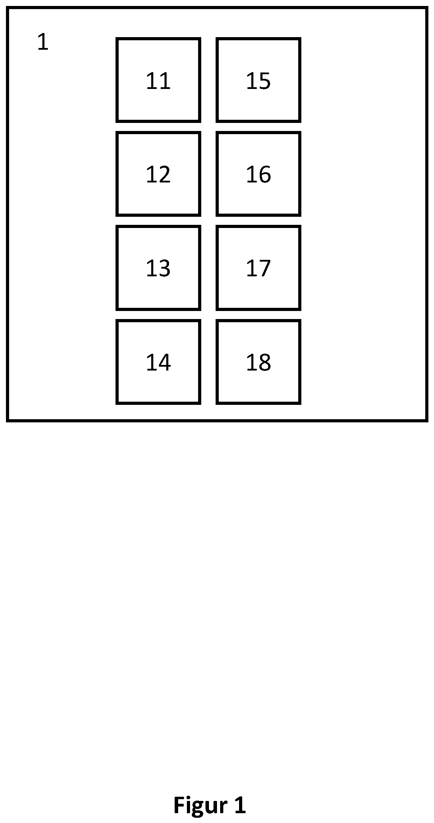

Im Folgenden wird das System anhand der Figuren 1 bis 7 über beispielhafte Ausführungsformen näher erläutert.

In Figur 1 ist beispielhaft ein Beatmungsgerät 1 mit einer Sensoreinheit 11, einer Aufbereitungseinheit 12, einer Berechnungseinheit 13, einer Erkennungseinheit 14, einer Speichereinheit 15, einer Überwachungseinheit 16, einer Steuereinheit 17 und einer Gebläse-/Ventileinheit 18 dargestellt. Die Einheiten 11, 12, 13, 14, 16, 17 können beispielweise Teil eines Computerprogramms sein, welches durch einen Prozessor auf dem Beatmungsgerät 1 ausgeführt wird. Auch eine Zusammenfassung der Einheiten 11, 12, 13, 14, 15, 16, 17 in einer Steuereinheit wäre beispielsweise denkbar.

Die Sensoreinheit 11 ist eingerichtet, um Messwerte, insbesondere Parameter, die mit einem Atemfluss, einem Atemvolumen, einer Atemfrequenz, einer Einatmungs- und Ausatmungsdauer, einer Atemkontur, einer Leckage oder einem Therapiedruck in Zusammenhang stehen, zu erfassen. Optional kann die Sensoreinheit 11 zusätzliche Messungen von Bestandteilen oder Temperatur des Atemgases oder des Blutes vornehmen. Die Sensoreinheit 11 übermittelt die erfassten Messwerte an die Aufbereitungseinheit 12.

Die Aufbereitungseinheit 12 kann die erfassten Messwerte aufbereiten. Beispielsweise kann die Aufbereitungseinheit 12 eine Glättung, eine Artefaktbereinigung oder ein Downsampling der Messwerte durchführen.

Die Berechnungseinheit 13 berechnet aus den durch die Sensoreinheit 11 erfassten und durch die Aufbereitungseinheit 12 aufbereiteten Messwerten Signale und/oder Kenngrößen, wie beispielsweise einen Mittelwert, einen Median, ein Perzentil, eine Ableitung, eine Häufigkeitsverteilung, eine Dauer oder einen Anteil eines Über- oder Unterschreitens von Schwellenwerten.

Das Erkennungseinheit 14 ist eingerichtet, um Ereignisse oder Zustände wie beispielsweise Alarme, Atemaussetzer, Artefakte, Hustenstöße, Sauerstoff(ent)sättigungen, Asynchronien 2 zwischen

Gerät und Anwender, verpasste Atemzüge 218, Auslöseverzögerungen 305, falsche Auslösungen 307, Einatmen, Ausatmen und/oder mandatorische Atemzüge zu erkennen.

Die Speichereinheit 15 speichert unter anderem die durch die Sensoreinheit 11 erfassten Werte oder Parameter

und/oder die durch die Aufbereitungseinheit 12 und/oder die Berechnungseinheit 13 aufbereiteten Werte, Daten und/oder Informationen, oder speichert diese zumindest zwischenzeitlich. Auch die durch die Erkennungseinheit 14 gewonnenen Informationen, Daten und Werte können und/oder werden in der Speichereinheit zumindest zwischengespeichert. Eine Zwischenspeicherung bedeutet zum Beispiel, dass die Werte, Daten und/oder Informationen bis zu einer Übertragung gespeichert und danach zum Beispiel gelöscht oder zum Überschreiben freigegeben werden.

Die Überwachungseinheit 16 erfasst zum Beispiel technische Probleme des Beatmungsgerätes 1. Technische Probleme können beispielsweise ein niedriger Akkustand, Fehler in der Elektronik, ein defekter Akku, ein defektes Bauteil, ein Stromausfall, ein nicht korrekt funktionierendes Zubehörteil, ein unplausibler Messwert oder ein Verlassen eines erlaubten Temperaturbereichs sein. Die Überwachungseinheit 17 kann bei einem erkannten technischen Problem einen Alarm auf dem Beatmungsgerät 1 über eine Schnittstelle anzeigen oder übermitteln.

Die Steuereinheit 17 dient beispielsweise zur Steuerung des Beatmungsgerätes 1, insbesondere einer Gebläse- und/oder Ventileinheit 18 zur Erzeugung des Atemgasstroms bzw. Beatmungsdruckes. Auch kann die Steuereinheit 17 ausgebildet sein, um andere Bestandteile und/oder Einheiten des Beatmungsgerätes 1 zu steuern. In manchen Ausführungsformen kann die Steuereinheit 17 auch weiter unterteilt sein und aus mehreren Steuereinheiten bestehen, welche jeweils eine individuelle Einheit und/oder einen Bestandteil des Beatmungsgerätes 1 steuern. Insbesondere ist die Steuereinheit 17 dazu eingerichtet, das Beatmungsgerät 1 anhand der ermittelten Daten, Werte und Erkenntnisse der Sensoreinheit 11, der Aufbereitungseinheit 12, der Berechnungseinheit 13 und/oder der Erkennungseinheit 14 zumindest teilweise automatisch zu steuern. In manchen Ausführungsformen ist die Steuerungseinheit 17 so eingerichtet, dass die Steuerung teilweise anhand manuell eingestellter Parameter und teilweise anhand automatisch eingestellter Parameter erfolgt. In manchen Ausführungsformen kann die Steuerung auch ausschließlich anhand manueller Einstellungen oder ausschließlich automatisch erfolgen.

Das Beatmungsgerät 1 ist beispielhaft dazu eingerichtet, einen gleichbleibenden Atemgasdruck vorzugeben (zum Beispiel in Form einer CPAP-Therapie) und/oder den vorgegebenen Atemgasdruck zwischen den Exspirationsphasen und Inspirationsphasen umzuschalten (zum Beispiel in Form eines Bi-Level-Beatmungsgerätes). Beispielsweise wird während der Inspirationsphase ein höherer Atemgasdruck vorgegeben als in der Exspirationsphase. In manchen Ausführungsformen findet eine Umschaltung zwischen Inspiration und Exspiration und/oder zwischen Exspiration und Inspiration in Form einer Druckrampe statt, sodass die Druck- und/oder Flussvorgabe nicht abrupt geändert wird. Statt über eine Druckrampe kann die Umschaltung auch in Form einer Flussrampe erfolgen. Der Punkt, zu welchem zwischen Inspiration und Exspiration umgeschaltet wird, kann beispielsweise zumindest teilweise über eine Auslöseempfindlichkeit bestimmt werden.

Die Erkennungseinheit 14 ist beispielsweise dazu eingerichtet, Asynchronien 2 zwischen dem Beatmungsgerät 1 und dem angeschlossenen Lebewesen zu erkennen. Eine Asynchronie 2 liegt zum Beispiel dann vor, wenn das Lebewesen einatmen möchte, das Beatmungsgerät 1 dies aber zum Beispiel nicht erkennt und keine Unterstützung während der Inspirationsphase auslöst. Auch der umgekehrte Fall, in dem das Lebewesen nicht einatmen möchte, aber das Beatmungsgerät 1 fälschlicherweise eine Anstrengung des Lebewesens zum Einatmen erkennt und entsprechend die Inspirationsunterstützung auslöst, kann als Asynchronie 2 zählen. Eine geplante, erzwungene Einatmung durch Auslösen der Inspirationsunterstützung durch das Beatmungsgerät 1, zum Beispiel weil das Lebewesen eine gewisse Zeit seit der letzten Inspiration überschreitet oder die maximale vorgegebene Zeit der Exspiration überschritten wurde, wird in den meisten Ausführungsformen nicht als Asynchronie 2 bewertet. Die Daten und Werte, die während einer erzwungenen Inspiration gemessen und/oder ermittelt werden, werden daher auch in der Regel nicht zur Bewertung der Asynchronien 2 herangezogen.

Die Erkennung der Asynchronien 2 wird beispielsweise während der Verwendung des Beatmungsgerätes 1 durch das Lebewesen durchgeführt. Dementsprechend werden die Ergebnisse der Erkennung unmittelbar "live" erzeugt und gegebenenfalls auch direkt durch das Beatmungsgerät 1, zum Beispiel zur Steuerung, verwendet.

Die in den Figuren 1 bis 3 gezeigte beispielhafte Ausführungsform des Systems erkennt dabei verpasste Atemzüge 218, also Ereignisse, bei denen das Lebewesen einatmen wollte, aber das Beatmungsgerät 1 keine Unterstützung bei der Inspiration ausgelöst hat, sowie kurze Auslöseverzögerungen 308, also Ereignisse, bei denen das Lebewesen nicht einatmen wollte, aber das Beatmungsgerät 1 eine Inspirationsunterstützung ausgelöst hat, als Asynchronien 2. Als kurze Auslöseverzögerungen 308 werden dabei die Auslöseverzögerungen 305, welche gleich oder kleiner als beispielsweise 0,1 sec bestimmt werden, bezeichnet. Bei diesen kurzen Auslöseverzögerungen 308 wird davon ausgegangen, dass das Lebewesen nicht einatmen wollte. In manchen Ausführungsformen ist das System auch so eingerichtet, dass Asynchronien 2 in Form von falschen Auslösungen 307 erkannt werden. Während die kurzen Auslöseverzögerungen 308 als ein Hinweis auf eine Auslösung ohne Absicht des Einatmens vom Lebewesen gesehen werden können, erkennt das System bei falschen Auslösungen 307 diese Ereignisse mit größerer Sicherheit.

Die Erkennungseinheit 14 erkennt die kurzen Auslöseverzögerungen 308 sowie die verpassten Atemzüge 218 aus dem Atemanstrengungsfluss, dem erwarteten Atemfluss sowie dem bestimmten Atemfluss. Der bestimmte Atemfluss stellt dabei den Atemfluss des Patienten dar, welcher aus dem vom Beatmungsgerät gemessenen Fluss unter Abzug der Leckage (vermutet, geschätzt oder gemessen) und anderen Ungenauigkeiten oder Einflüssen berechnet wird. Die

Berechnungen, welche die Grundlage der Erkennung der Asynchronien 2 durch die Erkennungseinheit 14 bilden, werden beispielsweise von der Berechnungseinheit 13 durchgeführt. Der erwartete Atemfluss wird aus dem effektiven Atemwegswiderstand R und der effektiven Lungenelastizität *E* bestimmt. Diese werden wiederum über ein mathematisches Lungenmodell, beispielsweise das Ein-Kompartment-Lungenmodell, berechnet. Die jeweiligen Parameter *R* und *E* können beispielsweise über multiple lineare Regression aus dem verwendeten Lungenmodell bestimmt werden.

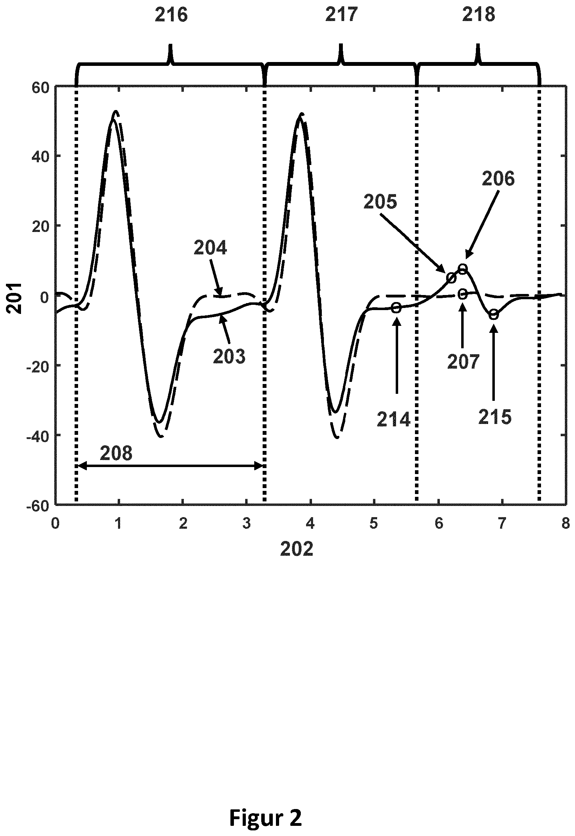

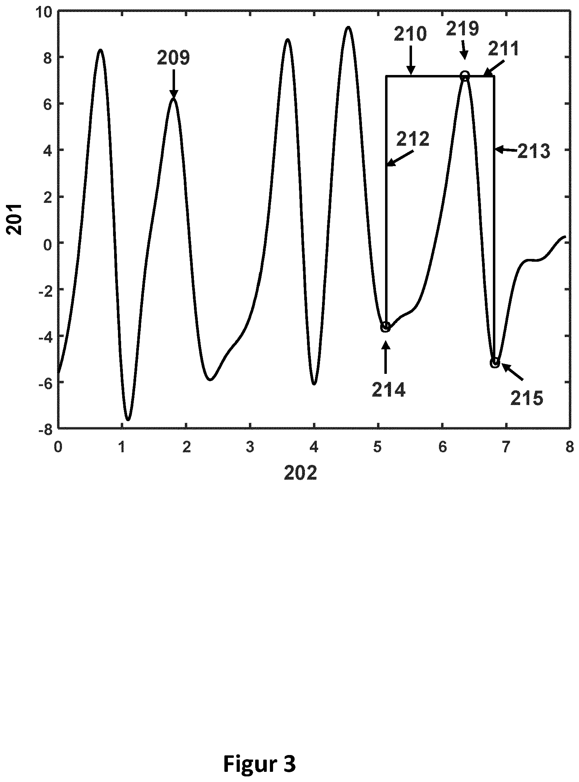

Die Erkennung der Asynchronien 2 in Form von verpassten Atemzügen 218 ist in den Figuren 2 und 3 schematisch dargestellt. In Figur 2 ist schematisch der zeitliche Verlauf des bestimmten Atemflusses 203 und des erwarteten Atemflusses 204 in einem Diagramm mit der Zeit 202 auf der x-Achse und der Flussrate 201 auf der y-Achse aufgetragen. Die Dauer eines Atemzuges entspricht dem Zeitabschnitt 208, wobei ein Atemzug grob in die Inspiration und Exspiration unterteilbar ist. Die Inspiration ist im Wesentlichen durch einen positiven Atemfluss zu erkennen. Die Exspiration ist im Wesentlichen durch einen negativen Atemfluss gekennzeichnet, wobei der Atemfluss zum Ende der Exspiration geringer - also in Richtung positiver Werte, aber dennoch negativ - wird und sich schließlich mit einer flacheren Steigung dem Wert Null annähert. In dem Diagramm der Figur 2b sind zwei vollständige Atemzüge 216, 217, zum Beispiel erkennbar an den positiven und negativen Peaks sowohl des bestimmten Atemflusses 203 als auch des erwarteten Atemflusses, dargestellt. Zudem ist auch ein verpasster Atemzug 218 beispielhaft dargestellt. Der verpasste Atemzug 218 ist beispielsweise daran zu erkennen, dass sowohl der positive als auch der negative Peak des bestimmten Atemflusses 203 deutlich kleinere Werte aufweisen als die der vollständigen Atemzüge 216, 217. Eine Unterstützung durch das Beatmungsgerät 1 wurde nicht ausgelöst, was beispielsweise an dem erwarteten Atemfluss 204 in Figur 2 zu sehen ist.

Die Erkennungseinheit 14 erkennt verpasste Atemzüge 218 zuverlässig anhand des Atemanstrengungsflusses 209, welcher in dem Diagramm in Figur 3 aufgetragen ist. Alternativ oder ergänzend kann vorgesehen sein, dass die verpassten Atemzüge 218 auch anhand von Merkmalen des Atemflusses und/oder des Drucks und/oder der Atemfrequenz und/oder des Atemvolumens erkannt werden. In dem Diagramm ist die Flussrate 201 (y-Achse) des Atemanstrengungsflusses gegen die Zeit 202 (x-Achse) aufgetragen. Die Erkennungseinheit 14 überprüft anhand der Werte des Atemanstrengungsflusses 209, des zeitlichen Verlaufs des Atemanstrengungsflusses 209, des bestimmten Atemflusses 203, des erwarteten Atemflusses 204 sowie der erwarteten Auslösezeitpunkte beispielsweise folgende Merkmale, um einen verpassten Atemzug 218 zu erkennen:

1. das lokale Maximum 206 des bestimmten Atemflusses 204 muss zwischen zwei Minima 214, 215 des Atemanstrengungsflusses 209 liegen;
2. Differenz 210 zwischen der zeitlichen Lage des lokalen Maximums 219 des Atemanstrengungsflusses und dem entsprechenden linken Minimum 214;
3. Differenz 211 zwischen der zeitlichen Lage des lokalen Maximums 219 des Atemanstrengungsflusses und dem entsprechenden rechten Minimum 215;
4. Differenz 212 zwischen den Werten des lokalen Maximums 219 des Atemanstrengungsflusses und dem entsprechenden linken Minimum 214;
5. Differenz 213 zwischen den Werten des lokalen Maximums 219 des Atemanstrengungsflusses und dem entsprechenden rechten Minimum 215;
6. erwarteter Atemfluss 207 zum Zeitpunkt des lokalen Maximums 219 des Atemanstrengungsflusses 209;
7. Zeit zwischen dem lokalen Maximum 219 des Atemanstrengungsflusses und dem erwarteten Auslösezeitpunkt 205.

Die Erkennungseinheit 14 ist beispielsweise so eingerichtet, dass diese Merkmale anhand eines Machine-Learning-Algorithmus überprüft werden. Beispielsweise werden dazu mehrere Daten von Lebewesen manuell ausgewertet und dem Machine-Learning-Algorithmus zur Verfügung gestellt, welcher daraus Werte, Daten, Parameter und Informationen ableitet, mit denen die verpassten Atemzüge 218 erkannt werden. Beispielsweise kann dazu ein Machine-Learning-Algorithmus auf Basis der Technik "AdaBoost M1" genutzt werden.

Die Erkennungseinheit 14 erkennt so beispielsweise anhand zumindest eines der Merkmale, dass ein verpasster Atemzug vorliegt. In manchen Ausführungsformen ist vorgesehen, dass mehrere, beispielsweise zumindest zwei, vier oder alle der Merkmale bei der Erkennung der verpassten Atemzüge genutzt werden bzw. die Kriterien erfüllen müssen, damit das jeweilige Merkmal auf einen verpassten Atemzug hindeutet.

In manchen Ausführungsformen kann vorgesehen sein, dass die Erkennung von verpassten Atemzügen durch die Verwendung von weiteren Parametern wie Drücken, Flüssen, Volumina, Frequenzen alternativ oder ergänzend durchgeführt und/oder weiter verfeinert wird.

Weiter ist die Erkennungseinheit 14 beispielhaft auch dazu eingerichtet, Auslöseverzögerungen 305 zu erkennen oder zu bestimmen und kurze Auslöseverzögerungen 308 als Asynchronie 2 zwischen dem Beatmungsgerät 1 und dem Lebewesen zu werten. Auslöseverzögerungen 305 werden beispielsweise für alle Atemzüge mit inspiratorischer Unterstützung bestimmt. Eine Überprüfung auf kurze Auslöseverzögerungen 308 wird zum Beispiel nur für spontane

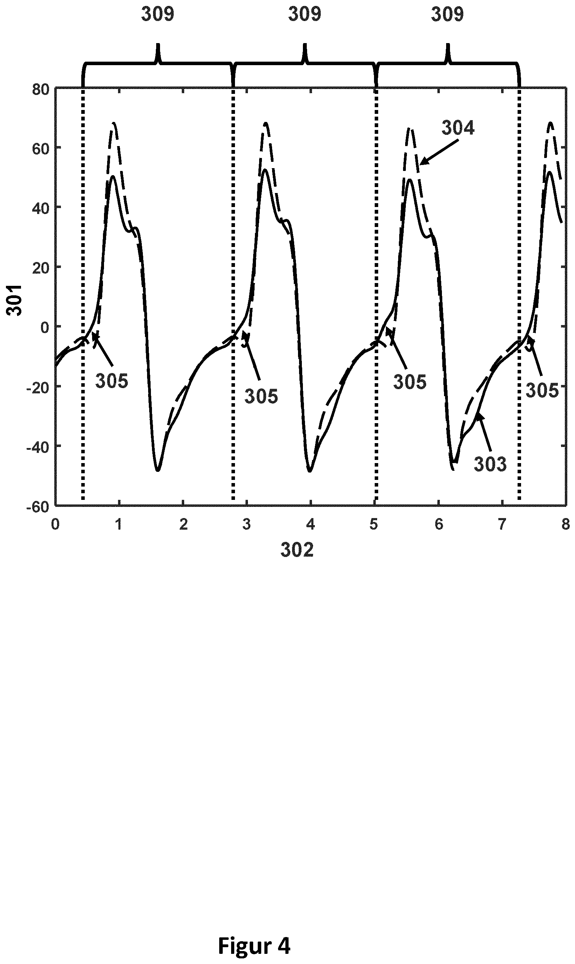

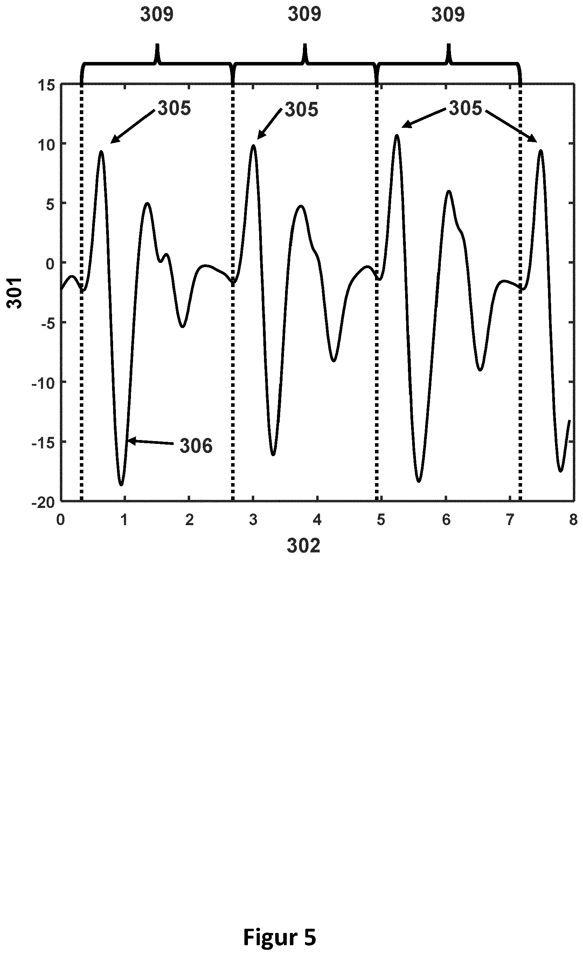

Atemzüge durchgeführt. Auslöseverzögerungen 305 sind beispielhaft in den Figuren 4 und 5 dargestellt. Figur 4 zeigt ein Diagramm, in dem die Flussraten 301 des bestimmten Atemflusses 303 und des erwarteten Atemflusses 304 gegen die Zeit 302 aufgetragen sind. Als Auslöseverzögerung 305 ist der Zeitabstand zwischen der Aktivierung der respiratorischen Muskeln des Lebewesens und der Auslösung der Atmungsunterstützung, beispielsweise zur Inspiration, durch das Beatmungsgerät 1 definiert. In Figur 4 sind die Auslöseverzögerungen 305 beispielsweise an dem Versatz zwischen dem Beginn der Steigung der Flussrate des bestimmten Atemflusses 303 und der erwarteten Flussrate 304 zu erkennen. Trägt man die Flussrate 301 des Atemanstrengungsflusses 305 gegen die Zeit 302 auf, wie in dem Diagramm in Figur 5 zu sehen, so können die Auslöseverzögerungen 305 an den lokalen Maxima der Atemzüge 309 erkannt werden.

Als kurze Auslöseverzögerungen 308 werden Auslöseverzögerungen 305 erkannt, deren Wert einen Schwellenwert von beispielsweise 0,1 sec nicht überschreiten. In manchen Ausführungsformen kann dieser Schwellenwert auch größer, beispielsweise bis zu 0,5 sec, oder kleiner, beispielsweise 0,05 sec, gewählt werden.

Die Auslöseverzögerungen 305 werden beispielsweise rechnerisch und über einen Algorithmus unter Verwendung des Atemanstrengungsflusses 305 bestimmt. Dazu wird ein Tiefpass-Filter, hier beispielsweise ein Butterworth-Filter dritter Ordnung, mit einer Grenzfrequenz von 3 Hz auf den Atemanstrengungsfluss 306 angewendet. Zudem wird unter Verwendung des Atemanstrengungsflusses 306 eine erwartete Amplitude A für den aktuellen Atemzug i berechnet. Für jeden Messpunkt k, beginnend unmittelbar vor dem Auslösepunkt der Inspiration und in die Exspirationsphase rückwärtsgehend, wird geprüft, ob dieser Messpunkt k Teil der Auslöseverzögerung ist, also zwischen Beginn der Atemanstrengung des Lebewesens und der Auslösung des Beatmungsgerätes 1 liegt. Ein Messpunkt k entspricht beispielsweise den durch die Sensoreinheit 11 zu einem Zeitpunkt aufgenommenen Messwerten, welche gegebenenfalls durch die Aufbereitungseinheit 12 und die Berechnungseinheit 13 weiterverarbeitet werden. Die Messpunkte k werden bezüglich des Atemflusses überprüft, ob sie einen bestimmten Schwellenwert, beispielsweise das $a_1$-fache der erwarteten Amplitude A, erreichen oder überschreiten, und als Anzahl $n_{highFlow}$ gezählt.

Der Faktor $a_1$ kann beispielsweise Werte zwischen 0,005 und 0,1 annehmen, bevorzugt zwischen 0,025 und 0,075. In manchen Ausführungsformen wird der Faktor $a_1$ auf einen Wert zwischen 0,05 und 0,06 festgelegt.

Ausgehend von dem letzten Messpunkt, welcher über dem Schwellenwert lag, wird zeitlich gesehen rückwärts die Anzahl $n_{increasingFlow}$ der Messpunkte gezählt, welche durch einen ansteigenden Atemanstrengungsfluss 306 gekennzeichnet sind und weitere Bedingungen erfüllen. Die Gesamtzahl der Messpunkte einer möglichen Auslöseverzögerung 305 entspricht der Summe n aus $n_{highFlow}$ und $n_{increaisngFlow}$. Weiter wird abschließend anhand der Messpunkte k geprüft, ob die Berechnung der Auslöseverzögerung 305 gültig ist. Sollte die Berechnung der Auslöseverzögerung 305 nicht gültig sein, so wird die Summe n als 0 gesetzt. Die Länge $t_{triggerDelay}$ der Auslöseverzögerung 305 ergibt sich aus der Multiplikation der Summe n mit einem Faktor $t_{tD}$. Der Faktor $t_{tD}$ liegt beispielsweise zwischen 0,001 sec und 0,05 sec, bevorzugt zwischen 0,005 sec und 0,015 sec. Liegt der Wert von $t_{triggerDelay}$ beispielsweise bei 0,1 sec oder weniger, so erkennt das Beatmungsgerät 1 durch die Erkennungseinheit 14 eine kurze Auslöseverzögerung 308, welche als Asynchronie 2 gewertet wird.

In manchen beispielhaften Ausführungsformen ist das Beatmungsgerät 1 mit der Erkennungseinheit 14 so eingerichtet, dass falsche Auslösungen 307 identifiziert werden können und diese als Asynchronie 2 erkannt werden. Dazu werden die Daten und Messwerte um den Auslösezeitpunkt $t_{trig}$ des Beatmungsgerätes 1 analysiert. Dazu werden die normalisierten Atemflüsse vor ($f_{pre}$) und nach ($f_{post}$) der Auslösung berechnet. Überschreiten die Werte für $f_{pre}$ und $f_{post}$ bestimmte, individuelle Schwellenwerte, wird eine korrekte Auslösung erkannt. Unterschreiten oder erreichen $f_{pre}$ und $f_{post}$ diese Werte, so wird eine falsche Auslösung 307 erkannt. Der Schwellenwert von $f_{pre}$ wird beispielsweise in einem Bereich von 0,005 und 0,5, bevorzugt zwischen 0,025 und 0,075, festgelegt. Der Schwellenwert für $f_{post}$ wird beispielsweise auf einen Wert zwischen 0,1 und 1,5, bevorzugt zwischen 0,5 und 1,0, festgelegt. Beispielsweise wird eine falsche Auslösung 307 erkannt, wenn $f_{pre} \leq 0{,}075$ und $f_{post} \leq 0{,}8$ gilt.

Anhand der erkannten Asynchronien 2 kann das Beatmungsgerät 1 die Auslöseempfindlichkeit 3 automatisch einstellen. In manchen Ausführungsformen kann eine Option für die automatische Einstellung der Auslöseempfindlichkeit 3 neben verschiedenen manuellen Stufen für die Auslöseempfindlichkeit 3 gewählt werden. Beispielsweise kann am Beatmungsgerät 1 für die Auslöseempfindlichkeit 3 eine Stufe von 1 bis 8 oder die automatische Auslöseempfindlichkeit 4 ausgewählt werden. Die Auslöseempfindlichkeit 3 gibt beispielsweise in einer abstrakten bzw. einheitslosen Zahl die Empfindlichkeit wieder, mit der das Beatmungsgerät 1 die Inspirationsunterstützung auslöst. Beispielsweise wird mit der Auslöseempfindlichkeit 3 zumindest ein Schwellenwert für den Atemfluss, bei dem die Inspirationsunterstützung durch das Beatmungsgerät 1 ausgelöst wird, berücksichtigt. In manchen beispielhaften Ausführungsformen hängt die Auslöseempfindlichkeit 3 hauptsächlich und/oder nur von einem Schwellenwert des Atemflusses ab. Eine höhere Stufe der Auslöseempfindlichkeit 3 bedeutet beispielsweise unter anderem einen höheren Schwellenwert für den Atemfluss, bei dem die Inspirationsunterstützung ausgelöst wird. Beispielsweise stellt Stufe 1 also eine empfindlichere Auslöseempfindlichkeit 3 als Stufe 2 dar.

Die Auslöseempfindlichkeit 3 kann beispielsweise über eine Benutzerschnittstelle, zum Beispiel eine als Touchscreen ausgeführte Anzeigeeinrichtung und/oder Eingabeeinrichtungen am Beatmungsgerät 1, eingestellt oder gewählt werden. Auch eine Einstellung oder Wahl der Auslöseempfindlichkeit 3 über eine räumlich von dem Beatmungs-

gerät 1 getrennte Gegenstelle ist möglich.

Wird die automatische Einstellung der Auslöseempfindlichkeit 3 ausgewählt, so stellt das Beatmungsgerät 1 die Auslöseempfindlichkeit 3 automatisch ein und berücksichtigt dabei mindestens die erkannten Asynchronien 2, beispielsweise verpasste Atemzüge 218 und kurze Auslöseverzögerungen 308. In manchen Ausführungsformen berücksichtigt das Beatmungsgerät 1 bei der automatischen Einstellung der Auslöseempfindlichkeit 3 zusätzlich erkannte falsche Auslösungen 307. Das Beatmungsgerät 1 kann die Auslöseempfindlichkeit 3 beispielsweise automatisch auf die Stufen 0 bis 3 einstellen. Die Stufe 0 stellt dabei eine noch empfindlichere Auslöseempfindlichkeit 3 als Stufe 1 dar. Die Stufe 0 ist beispielsweise nur für die automatische Einstellung verfügbar, kann manuell also nicht eingestellt werden. Es ist aber auch denkbar, dass alle Stufen sowohl für die manuelle als auch für die automatische Einstellung der Auslöseempfindlichkeit 3 verfügbar sind, also sowohl manuell als auch automatisch Stufen von 0 bis 8 eingestellt werden können. Neben einer Unterteilung in Stufen von 0 bis 8 kann die Auslöseempfindlichkeit 3 auch in eine andere, beliebige Anzahl an Stufen unterteilt werden. Diese können zum Beispiel durch Zahlen, Buchstaben oder Beschreibungen, zum Beispiel "sehr empfindlich", "empfindlich", "wenig empfindlich", "nicht empfindlich", gekennzeichnet werden. In manchen Ausführungsformen sind die Stufen der Auslöseempfindlichkeit 3 mit den Flusswerten, beispielsweise in l/min, angegeben. Die Auslöseempfindlichkeit 3 kann so beispielsweise zwischen 1 l/min bis 25 l/min oder 1 l/min bis 10 l/min einstellbar sein.

Die Auslöseempfindlichkeit 3 wird beispielsweise über den Parameter trigSens wiedergegeben. Bei einer manuellen Einstellung der Auslöseempfindlichkeit 3 wird dieser Parameter zur Einstellung beispielsweise direkt durch eine Eingabe über eine in Figur 1 nicht dargestellte Schnittstelle, beispielsweise eine Benutzerschnittstelle, verändert. Der Parameter trigSens wird bei der automatischen Einstellung je nach Anzahl der kurzen Auslöseverzögerungen 308 und verpassten Atemzüge 218 im aktuellen und letzten Zeitintervall bestimmt. Das Zeitintervall ist in der gezeigten beispielhaften Ausführungsform ein 2-Minuten-Intervall. Dieses Zeitintervall ist beispielsweise auf die vorhergehenden Zeitintervalle zur Bestimmung und/oder Erkennung der Asynchronien 2 angepasst. Die Werte des Parameters trigSens entsprechen dabei den Stufen der Auslöseempfindlichkeit 3.

Die Auslöseempfindlichkeit 3 wird über die Minimum-Funktion min(i,j) und/oder die Maximum-Funktion max(i,j) eingestellt. Die min()-Funktion liefert dabei den kleineren Wert von i und j - min(3,4) wäre also 3. Die max()-Funktion liefert wiederrum den größeren Wert von i und j - max(1,4) wäre dementsprechend 4.

Es wird ein Regelwerk aufgestellt, nach dem die Auslöseempfindlichkeit 3 angepasst wird. Beispielsweise folgende drei Regeln, welche sich gegenseitig ausschließen:

$$1.\ IF\ (\#ShoTrigDel \geq b\ \#IneffEff)\ \&\ (\#ShoTrigDel \geq c) \rightarrow trigSens = \min(trigSens + 1{,}3)$$

$$2.\ IF\ [(\#IneffEff \geq b\ \#ShoTrigDel)\ \&\ (\#IneffEff \geq c)] \rightarrow trigSens = \max(trigSens - 1{,}0)$$

$$3.\ IF\ (\#ShoTrigDel \leq d)\ \&\ (\#ShoTrigDel_{old} \leq d)\ \&\ (trigSens = trigSens_{old}) \rightarrow trigSens = \max(trigSens - 1{,}1)$$

#ShortTrigDel entspricht dabei der Anzahl an registrierten kurzen Auslöseverzögerungen 308 während des aktuellen Zeitintervalls, #IneffEff entspricht der Anzahl der registrierten verpassten Atemzüge 218 und trigSens stellt den aktuellen Wert oder die Stufe der Auslöseempfindlichkeit 3 dar. Der Index old deutet ferner auf die Werte des vorhergehenden Zeitintervalls dar. Dabei sind b, c und d Parameter in einem Bereich von 0 bis 6, beispielsweise in einem Bereich von 0 bis 3. In manchen Ausführungsformen können den Parametern b, c, d beispielsweise Werte von 0 bis 2 zugeordnet werden, wobei b und c den gleichen Wert haben können und d einen geringeren Wert, beispielsweise unter 1, aufweist.

In manchen Ausführungsformen können b, c und d auch andere Werte annehmen, dabei sollte aber darauf geachtet werden, dass sich die drei oben genannten Regeln weiterhin gegenseitig ausschließen, also immer nur eine Regel erfüllt wird oder aktiv ist. In manchen Ausführungsformen kann die dritte Regel auch so modifiziert werden, dass die Auslöseempfindlichkeit 3 (trigSens) nicht verändert wird, soweit keine Asynchronien 2 registriert werden, unabhängig davon, welche Stufe aktuell für die Auslöseempfindlichkeit 3 eingestellt ist.

Der Wert trigSens wird entsprechend der Minimum-Funktion min()oder der Maximum-Funktion max() geändert. Durch die Verwendung der Minimum- und Maximum-Funktion wird erreicht, dass bestimmte Werte - je nach Erfüllung der Regeln - nicht über- und/oder unterschritten werden können. In diesem Beispiel ist mit 0 bereits die kleinste mögliche Stufe eingesetzt. Wird in einer anderen Ausführungsform des Beatmungsgerätes 1 eine empfindlichere Stufe unter 0, beispielsweise -1, für die Auslöseempfindlichkeit definiert, so kann diese über die zweite Regel für die automatische Einstellung zugänglich gemacht werden.

Neben den gezeigten Regeln ist es zudem möglich, dass weitere Regeln definiert werden, anhand derer das Beatmungsgerät 1 die Auslöseempfindlichkeit 3 automatisch einstellen kann. Dabei wäre darauf zu achten, dass sich die definierten Regeln gegenseitig ausschließen. In manchen Ausführungsformen ist das Beatmungsgerät 1 oder die Erkennungseinheit 14 dazu eingerichtet, falsche Auslösungen 307 zu erkennen. Entsprechend können die bestehenden Regeln um die erkannten falschen Auslösungen 307 erweitert werden. Auch können beispielsweise zusätzliche Regeln definiert werden, welche die falschen Auslösungen 307 berücksichtigen. Diese können beispielsweise auch so formuliert sein, dass eine Erfüllung einer der ersten drei Regeln die Erfüllung zumindest einer der Regeln, welche die falschen Auslösungen 307 berücksichtigen, nicht ausschließt. Es kann also neben einer der ersten drei Regeln zumindest eine der weiteren Regeln erfüllt werden.

In manchen Ausführungsformen wird zum Beispiel davon ausgegangen, dass die Erkennung von Asynchronien 2 ungenauer wird, wenn die Leckageflüsse beispielsweise über einen Wert zwischen 15 l/min und 50 l/min, beispielsweise 25 l/min, steigen. Werden für ein Zeitintervall, beispielsweise ein 2-Minuten-Intervall, Leckageflüsse von 25 l/min oder höher vom Beatmungsgerät 1 erfasst, so ist das Beatmungsgerät 1 ausgebildet, um eine durchschnittliche Auslöseempfindlichkeit $\overline{T}_{new}$ für das nächste und/oder aktuelle Zeitintervall einzustellen. Diese durchschnittliche Auslöseempfindlichkeit $\overline{T}_{new}$ wird aus der durchschnittlichen Auslöseempfindlichkeit $\overline{T}_{old}$ des letzten Zeitintervalls sowie der berechneten aktuellen Auslöseempfindlichkeit T berechnet:

$$\overline{T}_{new} = (1 - \lambda_{Leak})\overline{T}_{old} + \lambda_{Leak} T$$

Der Faktor $\lambda_{leak}$ bezeichnet dabei einen Vergessensfaktor, welcher den Wert der alten durchschnittlichen Auslöseempfindlichkeit $\overline{T}_{old}$ gegenüber der aktuellen Auslöseempfindlichkeit T gewichtet. Beispielsweise wird $\lambda_{leak}$ ein Wert zwischen 0,01 und 0,9 zugeordnet, bevorzugt zwischen 0,1 und 0,5. Beispielsweise wird $\lambda_{leak}$ in der beispielhaften Ausführungsform der Wert 0,2 zugeordnet. In manchen Ausführungsformen wird die durchschnittliche Auslöseempfindlichkeit innerhalb von andauernden Phasen mit hohen Leckageflüssen über einem Schwellenwert von zum Beispiel 25 l/min nicht weiter angepasst. Das heißt, dass eine durchschnittliche Auslöseempfindlichkeit einmalig nach einem ersten Zeitintervall, beispielsweise 2 Minuten, hoher Leckageflüsse über 25 l/min neu berechnet wird und dann so lange beibehalten wird, wie kein Zeitintervall mit Leckageflüssen unter 25 l/min registriert wird.

In manchen Ausführungsformen des Systems wird während der Verwendungszeit, bei eingestellter manueller Auslöseempfindlichkeit 3, die Asynchronieerkennung durchgeführt und nach Beendigung der Verwendung eine Zusammenfassung über die erkannten Asynchronien 2 erstellt. Diese Zusammenfassung kann zudem Empfehlungen zur manuellen Einstellung der Auslöseempfindlichkeit 3 enthalten. Diese Empfehlungen können beispielsweise auf einer Anzeige (Display) des Beatmungsgerätes 1 oder über eine Schnittstelle zum Telemonitoring ausgegeben bzw. angezeigt werden.

Zudem ist die Generierung eines Alarms durch das Beatmungsgerät 1 denkbar, sollte ein Schwellenwert von erkannten verpassten Atemzügen 218, kurzen Auslöseverzögerungen 308 und/oder falschen Auslösungen 307 überschritten werden. Beispielsweise kann dieser Schwellenwert ein prozentualer Anteil der gesamten erkannten Atemzüge pro Zeitintervall sein. Das Zeitintervall kann dabei durchaus größer als die zur Asynchronieerkennung genutzten Zeitintervalle gewählt werden. Der prozentuale Anteil an erkannten Asynchronien, welcher zu einer Generierung eines Alarms führt, liegt beispielsweise zwischen 10 % und 100 %, in manchen Ausführungsformen zwischen 10 % und 50 %. Der Alarm kann beispielsweise über eine Schnittstelle, etwa ein Display oder eine Datenverbindung zum Telemonitoring, ausgegeben werden.

Die Erkennung von Asynchronien kann ferner verwendet werden, um zum Beispiel ungünstige Einstellungen des Beatmungsgerätes 1 und/oder einen intrinsischen PEEP (PEEP = positiver endexspiratorischer Druck) zu identifizieren. Ungünstige Einstellungen des Beatmungsgerätes 1 können beispielsweise in Druckeinstellungen und/oder Flusseinstellungen gefunden werden und bedeuten eine nicht optimale oder eine unzureichende Beatmung bzw. Unterstützung der Atmung des Lebewesens.

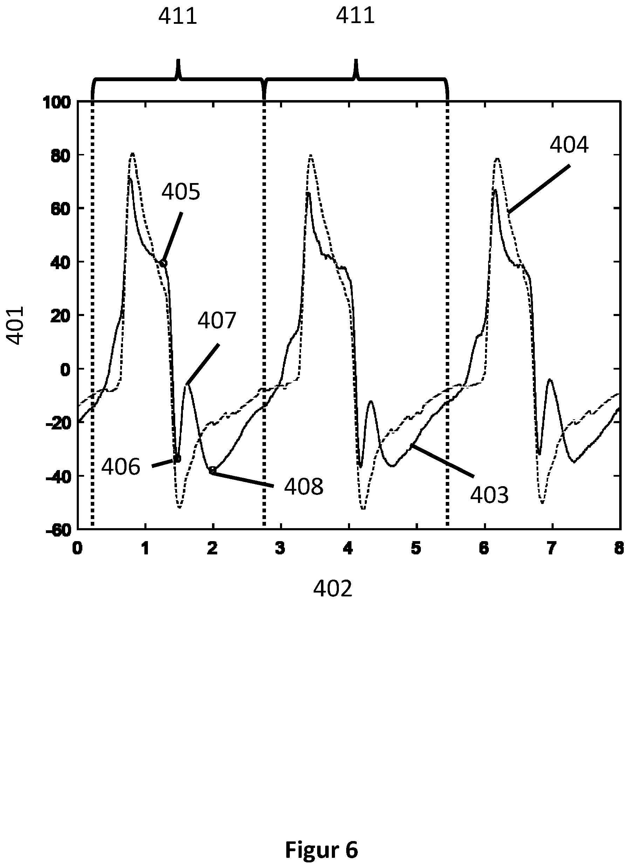

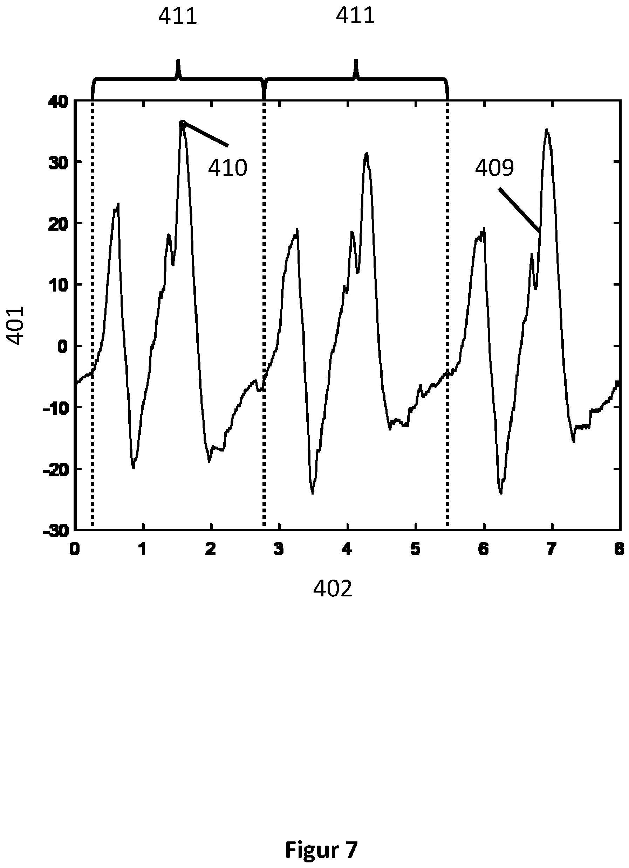

Die Figuren 6 und 7 zeigen beispielhaft die Erkennung von verfrühten Exspirationstriggern 412 über den Atemanstrengungsfluss 409 oder den bestimmten Atemfluss 403 und den erwarteten Atemfluss 404. In dem Diagramm in Figur 6 ist die Flussrate 401 des bestimmten Atemflusses 403 und des erwarteten Atemflusses 404 gegen die Zeit 402 aufgetragen. Abgebildet sind zwei volle Atemzüge 411. Die Flussrate 401 nimmt zu Beginn der Inspiration stark zu und flacht dann allmählich ab bzw. sinkt zum Ende der Inspiration wieder. Ab einer bestimmten Flussrate 401 wird beispielhaft eine Druckrampe gestartet (Beginn 405 der Druckrampe), über welche beispielsweise der durch das Beatmungsgerät 1 vorgegebene Druck verringert wird, um die Exspiration des Patienten zu unterstützen, zu ermöglichen und/oder herbeizuführen. Bei einer Exspiration entweicht Luft aus der Lunge, es wird also eine negative Flussrate 401 erwartet (erwarteter Atemfluss 404) und auch gemessen (gemessener Atemfluss 403). Nach Ende 406 der Druckrampe, mit welcher der Atemgasdruck durch das Beatmungsgerät 1 verringert wurde, sollte eine allmähliche Abflachung der Flussrate 401 zu beobachten sein, welche sich von einem negativen Peak (bzw. einem Minimum) wieder erhöht oder einer Flussrate 401

von 0 annähert. Ab einer bestimmten Flussrate 401 wird beispielsweise wieder eine Druckrampe durch das Beatmungsgerät 1 gestartet, über welche auf eine inspiratorische Druckunterstützung umgeschaltet wird.

Bei einem verfrühten Exspirationstrigger 412 wird die Druckrampe auf den exspiratorischen Druck zu früh gestartet. Der Patient oder Anwender ist zu dem Zeitpunkt noch nicht mit der Inspiration fertig, verlangt also noch nach Luft. Daher ist nach dem Ende 406 der Druckrampe auch ein erneuter starker Anstieg der Flussrate 401 des bestimmten Atemflusses 403 bis zu einem lokalen Maximum 407 zu erkennen. Nachfolgend passt sich der Patient an die Exspiration an, atmet also aus. Wie auch ohne verfrühte Exspirationstrigger 412 durchläuft die Flussrate 401 dabei auch ein lokales Minimum 408, bevor sich die Flussrate 401 wieder langsam erhöht.

Die Erkennungseinheit 14, gegebenenfalls in Kombination mit der Berechnungseinheit 13 und/oder der Aufbereitungseinheit 12 und/oder weiteren Komponenten des Beatmungsgerätes 1, ist beispielhaft dazu eingerichtet, anhand des Verlaufes des bestimmten Atemflusses 403 und des erwarteten Atemflusses 404 verfrühte Exspirationstrigger zu erkennen.

Beispielsweise werden dazu insbesondere vier Punkte überprüft: der Start 405 der Druckrampe von Inspiration zu Exspiration (durch das Beatmungsgerät 1), der Endpunkt 406 der Druckrampe, die Position des lokalen Maximums 407 des Atemflusses, welches nicht weiter als in einer Zeitspanne zwischen 0,3 Sekunden und 0,4 Sekunden nach dem Endpunkt 406 der Druckrampe lokalisiert sein sollte, sowie des lokalen Minimums 408, welches innerhalb von einer Zeitspanne zwischen 0,5 Sekunden und 0,7 Sekunden dem Endpunkt 406 der Druckrampe folgen sollte.

Ein verfrühter Exspirationstrigger wird in der beispielhaften Ausführungsform beispielsweise detektiert, wenn zumindest eine der folgenden Bedingungen zutrifft:

1. der bestimmte Atemfluss 403 liegt zu Beginn 405 der Druckrampe über 0 l/min;
2. der bestimmte Atemfluss 403 liegt zum Ende 405 der Druckrampe unter 0 l/min;
3. e * bestimmter Atemfluss 403 (am lokalen Maximum 407) / bestimmter Atemfluss 403 (zum Ende 406 der Druckrampe) < erwarteter Atemfluss 404 (Maximum) / erwarteter Atemfluss 403 (zum Ende der Druckrampe);
4. f * bestimmter Atemfluss 403 (am lokalen Maximum 407) > bestimmter Atemfluss 403 (zum Ende 406 der Druckrampe);
5. g * bestimmter Atemfluss 403 (am lokalen Minimum 408) < bestimmter Atemfluss 403 (zum Ende 406 der Druckrampe) + bestimmter Atemfluss 403 (am lokalen Maximum 407);
6. (h < bestimmter Atemfluss 403 (am lokalen Maximum 407) - bestimmter Atemfluss 403 (zum Ende 406 der Druckrampe)) ODER (h < bestimmter Atemfluss 403 (lokales Maximum 407) - bestimmter Atemfluss 403 (am lokalen Minimum 408));
7. bestimmter Atemfluss 403 (am lokalen Minimum 408) < 0.

Dabei liegt der Faktor e in einem Bereich von 0,5 bis 2,0, bevorzugt zwischen 1 und 1,8, der Faktor f in einem Bereich von 0,9 bis 2,9, bevorzugt zwischen 1,7 und 2,3, der Faktor g in einem Bereich von 1 bis 3, bevorzugt zwischen 1,8 und 2,2, und h in einem Bereich von 2 l/min bis 10 l/min, bevorzugt zwischen 3 l/min und 7 l/min.

In manchen Ausführungsformen ist vorgesehen, dass zumindest 2 oder 4 oder alle der Bedingungen zutreffen müssen, damit ein verfrühter Exspirationstrigger detektiert wird.

Auch in dem Verlauf des Atemanstrengungsflusses 409 lässt sich ein verfrühter Exspirationstrigger 412 erkennen, wie in Figur 7 dargestellt. Zu sehen sind die gleichen Atemzüge 411 wie in Figur 6, hier als Verlauf der Flussrate 401 des Atemanstrengungsflusses 409 mit der Zeit 402. Durch das lokale Maximum 407 des bestimmten Atemflusses 403 ist ebenso ein lokales Maximum 410 im Atemanstrengungsfluss 409 zu sehen. Der erwartete Atemfluss 404 sieht keinen direkten Anstieg der Flussrate 401 nach Ende 406 der Druckrampe vor, daher entsteht hier ein großer Unterschied zum bestimmten Atemfluss 403, welcher sich in dem stark positiven lokalen Maximum 410 des Atemanstrengungsflusses 409 niederschlägt. In manchen Ausführungsformen kann beispielsweise über die Höhe des lokalen Maximums 410 und auch die Lage in Bezug auf das Ende 406 der Druckrampe eine Aussage über verfrühte Exspirationstrigger getroffen werden. Beispielsweise kann anhand der Höhe des lokalen Maximums 410 eine Änderung der Auslöseempfindlichkeit 3 gesteuert werden. In manchen Ausführungsformen ist zum Beispiel denkbar, dass die Auslöseempfindlichkeit 3 anhand hinterlegter Werte für die Höhe des lokalen Maximums 410 angepasst wird.

Das System ist in manchen Ausführungsformen darauf ausgelegt, anhand der verfrühten Exspirationstrigger 412 die Auslöseempfindlichkeit 3 anzupassen. Beispielsweise kann eine solche Anpassung automatisch erfolgen. Wird innerhalb eines (ggf. einstellbaren) Zeitraums und/oder einer (ggf. einstellbaren) Anzahl an Atemzügen 411 eine gewisse Anzahl an verfrühten Exspirationstriggern 412 detektiert, kann das Beatmungsgerät 1 beispielsweise ausgebildet sein, um zu erkennen, dass eine zu sensitive Auslöseempfindlichkeit 3 für die Umschaltung von Inspiration auf Exspiration vorliegt. Beispielhaft wird dann die Auslöseempfindlichkeit 3, zumindest für die Umschaltung von Inspiration auf Exspiration, verringert, also auf einen weniger sensitiven Wert eingestellt.

Die (automatische) Einstellung der Auslöseempfindlichkeit 3 auf Basis verpasster Atemzüge 218 und/oder kurzer Auslöseverzögerungen 305 und/oder falscher Auslösungen 307 bezieht sich beispielsweise insbesondere auf

einen (Schwellen-)Wert, welcher die Schaltung auf eine Inspirationsunterstützung (z. B. Umschalten von Exspiration auf Inspiration) betrifft. Werden verfrühte Exspirationstrigger 412 mit in die Einstellung einbezogen, so betreffen diese beispielweise insbesondere (Schwellen-)Werte, anhand derer eine Umschaltung von Inspiration auf Exspiration erfolgt.

Bezugszeichenliste

| | |
|---|---|
| 1 | Beatmungsgerät |
| 2 | Asynchronie |
| 3 | Auslöseempfindlichkeit |
| 4 | automatische Auslöseempfindlichkeit |
| 11 | Sensoreinheit |
| 12 | Aufbereitungseinheit |
| 13 | Berechnungseinheit |
| 14 | Erkennungseinheit |
| 15 | Speichereinheit |
| 16 | Überwachungseinheit |
| 17 | Steuereinheit |
| 18 | Gebläse-/Ventileinheit |
| 201 | Flussrate (Flow) (y-Achse) |
| 202 | Zeit (x-Achse) |
| 203 | bestimmter Atemfluss |
| 204 | erwarteter Atemfluss |
| 205 | erwarteter Auslösepunkt |
| 206 | lokales Maximum |
| 207 | erwarteter Atemfluss |
| 208 | Atemzugdauer |
| 209 | Atemanstrengungsfluss |
| 210 | Differenz (links) |
| 211 | Differenz (rechts) |
| 212 | Differenz (links) |
| 213 | Differenz (rechts) |
| 214 | Minimum (links) |
| 215 | Minimum (rechts) |
| 216 | Atemzug |
| 217 | Atemzug |
| 218 | verpasster Atemzug |
| 219 | lokales Maximum |
| 301 | Flussrate (Flow) (y-Achse) |
| 302 | Zeit (x-Achse) |
| 303 | bestimmter Atemfluss |
| 304 | erwarteter Atemfluss |
| 305 | Auslöseverzögerung |
| 306 | Atemanstrengungsfluss |
| 307 | falsche Auslösung |
| 308 | kurze Auslöseverzögerung |
| 309 | Atemzug |
| 401 | Flussrate (Flow) (y-Achse) |
| 402 | Zeit (x-Achse) |
| 403 | bestimmter Atemfluss |
| 404 | erwarteter Atemfluss |
| 405 | Startpunkt (Druckrampe) |
| 406 | Endpunkt (Druckrampe) |
| 407 | lokales Maximum |
| 408 | lokales Minimum |
| 409 | Atemanstrengungsfluss |
| 410 | lokales Maximum |

411 Atemzug
412 verfrühter Exspirationstrigger

**Patentansprüche**

1. System zur Erkennung von Asynchronien zwischen einem Beatmungsgerät (1) und einem Lebewesen, wobei das System zumindest ein Beatmungsgerät (1) umfasst, wobei das Beatmungsgerät (1) zumindest umfasst:

   eine Sensoreinheit (11);
   eine Aufbereitungseinheit (12);
   eine Berechnungseinheit (13);
   eine Erkennungseinheit (14), die ausgebildet ist, um die Asynchronien (2) zwischen dem Beatmungsgerät (1) und dem Lebewesen anhand von Atemparametern des Lebewesens zu erkennen;
   eine Speichereinheit (15);
   eine Überwachungseinheit (16);
   eine Gebläse- und/oder Ventileinheit (18); und
   eine Steuereinheit (17), die ausgebildet ist, um zumindest anhand der durch die Erkennungseinheit erkannten Asynchronien das Beatmungsgerät (1) durch Steuern der Gebläse- und/oder Ventileinheit (18) zumindest teilweise und zumindest zeitweise automatisch zu steuern;
   **dadurch gekennzeichnet, dass** die Berechnungseinheit (13) ausgebildet ist, um einen Atemwegswiderstand R und eine Lungenelastizität E aus durch die Sensoreinheit (11) gemessenen und durch die Aufbereitungseinheit (12) aufbereiteten Messwerten über ein mathematisches Lungenmodell zu berechnen, einen erwarteten Atemfluss (204, 304) aus dem Atemwegswiderstand R und der Lungenelastizität E zu bestimmen und einen Atemanstrengungsfluss (209, 306) als eine Abweichung des erwarteten Atemflusses (204, 304) von einem bestimmten Atemfluss (203, 303) zu bestimmen, wobei der bestimmte Atemfluss (203, 303) einen aus einem vom Beatmungsgerät (1) gemessenen Fluss unter Abzug einer Leckage und anderen Einflüssen berechneten Atemfluss des Patienten darstellt, wobei die Erkennungseinheit (14) ferner ausgebildet ist, um verpasste Atemzüge (218) durch Auswerten des Atemanstrengungsflusses (209, 306), des erwarteten Atemflusses (204, 304) und des bestimmten Atemflusses (203, 303) zu erkennen und als die Asynchronien (2) zu bewerten, wobei die verpassten Atemzüge (218) jene Atemzüge sind, bei denen das Lebewesen zwar eine Atemanstrengung zeigt, aber die Atemanstrengung nicht durch das Beatmungsgerät (1) erkannt wird, sodass keine inspiratorische Unterstützung erfolgt, wobei die Erkennungseinheit (14) die verpassten Atemzüge (218) anhand von zumindest zwei der folgenden Erkennungsmerkmale des Atemanstrengungsflusses (209, 306), des erwarteten Atemflusses (204, 304) und des bestimmten Atemflusses (203, 303) erkennt:

   - das lokale Maximum (206) des bestimmten Atemflusses (204) liegt zwischen zwei Minima (214, 215) des Atemanstrengungsflusses (209, 306);
   - Differenz (210) zwischen der zeitlichen Lage des lokalen Maximums (219) des Atemanstrengungsflusses (209, 306) und dem entsprechenden linken Minimum (214);
   - Differenz (211) zwischen der zeitlichen Lage des lokalen Maximums (219) des Atemanstrengungsflusses (209, 306) und dem entsprechenden rechten Minimum (215);
   - Differenz (212) zwischen den Werten des lokalen Maximums (219) des Atemanstrengungsflusses (209, 306) und dem entsprechenden linken Minimum (214);
   - Differenz (213) zwischen den Werten des lokalen Maximums (219) des Atemanstrengungsflusses (209, 306) und dem entsprechenden rechten Minimum (215);
   - erwarteter Atemfluss (207) zum Zeitpunkt des lokalen Maximums (219) des Atemanstrengungsflusses (209, 306);
   - Zeit zwischen dem lokalen Maximum (219) des Atemanstrengungsflusses (209, 306) und einem erwarteten Auslösezeitpunkt (205) des Beatmungsgerätes (1).

2. System nach Anspruch 1,
   wobei die Erkennungseinheit (14) die verpassten Atemzüge (218) anhand von zumindest vier oder aller der Erkennungsmerkmale erkennt.

3. System nach einem der vorhergehenden Ansprüche,
   wobei die Erkennungseinheit (14) ferner ausgebildet ist, um kurze Auslöseverzögerungen (308) und/oder falsche Auslösungen (307) und/oder verfrühte Exspirationstrigger (412) zu erkennen und als die Asynchronien (2) zu bewerten.

4. System nach Anspruch 3,
wobei die Erkennungseinheit (14) ferner ausgebildet ist, um die kurzen Auslöseverzögerungen (308) durch Auswerten des Atemanstrengungsflusses (209, 306), des erwarteten Atemflusses (204, 304) und des bestimmten Atemflusses (203, 303) zu erkennen.

5. System nach Anspruch 3 oder 4,
wobei die Steuereinheit (17) ausgebildet ist, um anhand der von der Erkennungseinheit (14) erkannten verpassten Atemzüge (218) und kurzen Auslöseverzögerungen (308) eine Auslöseempfindlichkeit (3) des Beatmungsgerätes (1) automatisch einzustellen.

6. System nach Anspruch 5,
wobei die Steuereinheit (17) ausgebildet ist, um ferner anhand der von der Erkennungseinheit (14) erkannten falschen Auslösungen (307) die Auslöseempfindlichkeit (3) des Beatmungsgerätes (1) automatisch einzustellen.

7. System nach Anspruch 5 oder 6,
wobei die Steuereinheit (17) ausgebildet ist, um die Auslöseempfindlichkeit (3) entsprechend der Anzahl der erkannten kurzen Auslöseverzögerungen (308) und verpassten Atemzüge (218) innerhalb eines Zeitintervalls zwischen 0,5 und 5 Minuten, bevorzugt zwischen 1 und 3 Minuten, automatisch anzupassen.

8. System nach einem der Ansprüche 5 bis 7,
wobei bei Leckageflüssen über einem Schwellenwert zwischen 15 l/min und 50 l/min, insbesondere von 25 l/min, die Auslöseempfindlichkeit (3) in Form einer durchschnittlichen Auslöseempfindlichkeit eingestellt wird, die unter Einbeziehung von Auslöseempfindlichkeiten (3) aus vorherigen Zeiträumen mit Leckageflüssen unter dem Schwellenwert bestimmt wird.

9. System nach einem der Ansprüche 5 bis 8,
wobei die Auslöseempfindlichkeit (3) manuell und automatisch einstellbar ist, wobei die automatische Einstellung der Auslöseempfindlichkeit (3) geringere Schwellenwerte der Parameter zur Auslösung einstellen kann, als durch die manuelle Einstellung möglich ist.

10. System nach einem der Ansprüche 5 bis 9,
wobei die Auslöseempfindlichkeit (3) einen Wert umfasst, der eine Umschaltung von einer Inspirationsphase auf eine Exspirationsphase steuert, wobei das Beatmungsgerät (1) ausgebildet ist, um anhand der erkannten verfrühten Exspirationstrigger (412) den Wert der Auslöseempfindlichkeit (3), der die Umschaltung von einer Inspirationsphase auf eine Exspirationsphase steuert, einzustellen.

11. System nach einem der Ansprüche 3 bis 10,
wobei eine Auslöseverzögerung (305) als eine kurze Auslöseverzögerung (308) erkannt wird, wenn die Auslöseverzögerung (305, 308) kleiner oder gleich einem Schwellenwert ist, wobei der Schwellenwert in einem Bereich zwischen 0 Sekunden und 0,5 Sekunden, bevorzugt zwischen 0 Sekunden und 0,25 Sekunden, besonders bevorzugt zwischen 0 Sekunden und 0,15 Sekunden, gewählt wird.

12. System nach Anspruch 11,
wobei die Erkennungseinheit (14) ausgebildet ist, um die Auslöseverzögerung (305) über einen Versatz zwischen der Atemanstrengung des Lebewesens und der Auslösung des Beatmungsgerätes (1) zu erkennen und zu bestimmen.

13. System nach einem der Ansprüche 3 bis 12,
wobei das Beatmungsgerät (1) ausgebildet ist, um die verfrühten Exspirationstrigger (412) anhand eines zeitlichen Verlaufs des bestimmten Atemflusses (403) und/oder des erwarteten Atemflusses (404) und/oder des Atemanstrengungsflusses (409) zu erkennen.

14. System nach einem der Ansprüche 3 bis 13,
wobei das Beatmungsgerät (1) ausgebildet ist, um die verfrühten Exspirationstrigger (412) anhand einer Lage und eines Werts einer Flussrate eines Starts (405) einer Druckrampe, eines Endes (406) einer Druckrampe, eines lokalen Maximums (407, 410) und/oder eines lokalen Minimums (408) des bestimmten Atemflusses (403) und/oder des erwarteten Atemflusses (404) zu erkennen.

15. System nach einem der Ansprüche 3 bis 14,

wobei das Beatmungsgerät (1) ausgebildet ist, um die verfrühten Exspirationstrigger (412) zu erkennen, wenn zumindest eine der folgenden Bedingungen zutrifft:

- der bestimmte Atemfluss (403) liegt zu Beginn (405) einer Druckrampe über 0 l/min;
- der bestimmte Atemfluss (403) liegt zum Ende (405) einer Druckrampe unter 0 l/min;
- e * bestimmter Atemfluss (403) am lokalen Maximum (407) / bestimmter Atemfluss (403) zum Ende (406) einer Druckrampe < erwarteter Atemfluss (404) / erwarteter Atemfluss (403) zum Ende der Druckrampe;
- f * bestimmter Atemfluss (403) am lokalen Maximum (407) > bestimmter Atemfluss (403) zum Ende (406) einer Druckrampe;
- g * bestimmter Atemfluss (403) am lokalen Minimum (408) < bestimmter Atemfluss (403) zum Ende (406) einer Druckrampe + bestimmter Atemfluss (403) am lokalen Maximum (407);
- (h < bestimmter Atemfluss (403) am lokalen Maximum (407) - bestimmter Atemfluss (403) zum Ende (406) der Druckrampe) ODER (h < bestimmter Atemfluss (403) am lokalen Maximum (407) - bestimmter Atemfluss (403) am lokalen Minimum (408));
- bestimmter Atemfluss (403) am lokalen Minimum (408) < 0;

wobei der Faktor e in einem Bereich von 0,5 bis 2,0, bevorzugt zwischen 1 und 1,8, liegt, wobei der Faktor f in einem Bereich von 0,9 bis 2,9, bevorzugt zwischen 1,7 und 2,3, liegt, wobei der Faktor g in einem Bereich von 1 bis 3, bevorzugt zwischen 1,8 und 2,2, liegt und wobei h in einem Bereich von 2 l/min bis 10 l/min, bevorzugt zwischen 3 l/min und 7 l/min, liegt.

**16.** System nach einem der vorhergehenden Ansprüche, wobei der Atemwegswiderstand R und die Lungenelastizität E durch die Berechnungseinheit (13) über eine multiple lineare Regression und das Ein-Kompartment-Lungenmodell bestimmt werden.

## Claims

**1.** A system for detecting asynchrony between a ventilator (1) and a living being, wherein the system comprises at least one ventilator (1), wherein the ventilator (1) comprises at least:

a sensor unit (11);
a processing unit (12);
a calculation unit (13);
a detection unit (14) designed to detect the asynchrony (2) between the ventilator (1) and the living being on the basis of respiratory parameters of the living being;
a memory unit (15);
a monitoring unit (16);
a blower and/or valve unit (18); and
a control unit (17) designed to automatically control the ventilator (1), at least partially and at least temporarily and at least on the basis of the asynchrony detected by the detection unit, by controlling the blower and/or valve unit (18);
**characterized in that** the calculation unit (13) is designed to calculate an airway resistance R and a lung elasticity E from measurement values measured by the sensor unit (11) and processed by the processing unit (12) using a mathematical lung model, to determine an expected respiratory flow (204, 304) from the airway resistance R and the lung elasticity E, and to determine a respiratory effort flow (209, 306) as a deviation of the expected respiratory flow (204, 304) from a determined respiratory flow (203, 303), wherein the determined respiratory flow (203, 303) represents a respiratory flow of the patient calculated from a flow measured by the ventilator (1) with leakage and other influences subtracted, wherein the detection unit (14) is further designed to detect missed breaths (218) by evaluating the respiratory effort flow (209, 306), the expected respiratory flow (204, 304), and the determined respiratory flow (203, 303) and to assess them as the asynchrony (2), wherein the missed breaths (218) are those breaths in which the living being does exhibit a respiratory effort but the respiratory effort is not detected by the ventilator (1) such that no inspiratory support occurs, wherein the detection unit (14) detects the missed breaths (218) on the basis of at least two of the following detection characteristics of the respiratory effort flow (209, 306), the expected respiratory flow (204, 304), and the determined respiratory flow (203, 303):

- the local maximum (206) of the determined respiratory flow (204) lies between two minima (214, 215) of the respiratory effort flow (209, 306);

- the difference (210) between the temporal position of the local maximum (219) of the respiratory effort flow (209, 306) and the corresponding left-hand minimum (214);
- the difference (211) between the temporal position of the local maximum (219) of the respiratory effort flow (209, 306) and the corresponding right-hand minimum (215);
- the difference (212) between the values of the local maximum (219) of the respiratory effort flow (209, 306) and the corresponding left-hand minimum (214);
- the difference (213) between the values of the local maximum (219) of the respiratory effort flow (209, 306) and the corresponding right-hand minimum (215);
- expected respiratory flow (207) at the time of the local maximum (219) of the respiratory effort flow (209, 306);
- time between the local maximum (219) of the respiratory effort flow (209, 306) and an expected trigger time (205) of the ventilator (1).

**2.** The system according to claim 1,
wherein the detection unit (14) detects the missed breaths (218) on the basis of at least four or all of the detection characteristics.

**3.** The system according to one of the preceding claims,
wherein the detection unit (14) is further designed to detect short trigger delays (308) and/or false triggers (307) and/or premature expiratory triggers (412) and to assess them as the asynchrony (2).

**4.** The system according to claim 3,
wherein the detection unit (14) is further designed to detect the short trigger delays (308) by evaluating the respiratory effort flow (209, 306), the expected respiratory flow (204, 304), and the determined respiratory flow (203, 303).

**5.** The system according to claim 3 or 4,
wherein the control unit (17) is designed to automatically set a trigger sensitivity (3) of the ventilator (1) on the basis of the missed breaths (218) and short trigger delays (308) detected by the detection unit (14).

**6.** The system according to claim 5,
wherein the control unit (17) is further designed to automatically set the trigger sensitivity (3) of the ventilator (1) on the basis of the false triggers (307) detected by the detection unit (14).

**7.** The system according to claim 5 or 6,
wherein the control unit (17) is designed to automatically adjust the trigger sensitivity (3) depending on the number of detected short trigger delays (308) and missed breaths (218) within a time interval between 0.5 and 5 minutes, preferably between 1 and 3 minutes.

**8.** The system according to one of claims 5 to 7,
wherein, for leakage flows exceeding a threshold between 15 l/min and 50 l/min, in particular of 25 l/min, the trigger sensitivity (3) is set in the form of an average trigger sensitivity that is determined by taking into account trigger sensitivities (3) from previous time periods having leakage flows below the threshold.

**9.** The system according to one of claims 5 to 8,
wherein the trigger sensitivity (3) can be set both manually and automatically, wherein the automatic setting of the trigger sensitivity (3) can set lower thresholds of the triggering parameters than are possible with the manual setting.

**10.** The system according to one of claims 5 to 9,
wherein the trigger sensitivity (3) comprises a value that controls switching from an inspiratory phase to an expiratory phase, wherein the ventilator (1) is configured to set, based on the detected premature expiratory triggers (412), the value of the trigger sensitivity (3) that controls the switching from an inspiratory phase to an expiratory phase.

**11.** The system according to one of claims 3 to 10,
wherein a trigger delay (305) is detected as a short trigger delay (308) when the trigger delay (305, 308) is less than or equal to a threshold value, wherein the threshold value is selected in a range between 0 seconds and 0.5 seconds, preferably between 0 seconds and 0.25 seconds, and particularly preferably between 0 seconds and 0.15 seconds.

**12.** The system according to claim 11,

wherein the detection unit (14) is designed to detect and determine the trigger delay (305) based on an offset between the respiratory effort of the living being and the triggering of the ventilator (1).

**13.** The system according to one of claims 3 to 12, wherein the ventilator (1) is designed to detect the premature expiratory triggers (412) on the basis of a temporal progression of the determined respiratory flow (403) and/or of the expected respiratory flow (404) and/or of the respiratory effort flow (409).

**14.** The system according to one of claims 3 to 13, wherein the ventilator (1) is designed to detect the premature expiratory triggers (412) on the basis of a position and a value of a flow rate at a start (405) of a pressure ramp, at an end (406) of a pressure ramp, at a local maximum (407, 410) and/or at a local minimum (408) of the determined respiratory flow (403) and/or of the expected respiratory flow (404).

**15.** The system according to one of claims 3 to 14,

wherein the ventilator (1) is designed to detect the premature expiratory triggers (412) when at least one of the following conditions is met:

- the determined respiratory flow (403) is greater than 0 l/min at the beginning (405) of a pressure ramp;
- the determined respiratory flow (403) is below 0 l/min at the end (405) of a pressure ramp;
- e * determined respiratory flow (403) at the local maximum (407) / determined respiratory flow (403) at the end (406) of a pressure ramp < expected respiratory flow (404) / expected respiratory flow (403) at the end of the pressure ramp;
- f * determined respiratory flow (403) at the local maximum (407) > determined respiratory flow (403) at the end (406) of a pressure ramp;
- g * determined respiratory flow (403) at the local minimum (408) < determined respiratory flow (403) at the end (406) of a pressure ramp + determined respiratory flow (403) at the local maximum (407);
- (h < determined respiratory flow (403) at the local maximum (407) - determined respiratory flow (403) at the end (406) of the pressure ramp) OR (h < determined respiratory flow (403) at the local maximum (407) - determined respiratory flow (403) at the local minimum (408));
- determined respiratory flow (403) at the local minimum (408) < 0;

wherein the factor e is in a range from 0.5 to 2.0, preferably between 1 and 1.8, wherein the factor f is in a range from 0.9 to 2.9, preferably between 1.7 and 2.3, wherein the factor g is in a range from 1 to 3, preferably between 1.8 and 2.2, and wherein h is in a range from 2 l/min to 10 l/min, preferably between 3 l/min and 7 l/min.

**16.** The system according to one of the preceding claims, wherein the airway resistance R and the lung elasticity E are determined by the calculation unit (13) using multiple linear regression and the single-compartment lung model.

## Revendications

**1.** Système de détection d'asynchronies entre un respirateur (1) et un être vivant, ledit système comprenant au moins un respirateur (1), ledit respirateur (1) comprenant au moins :

une unité de capteurs (11) ;
une unité de traitement (12) ;
une unité de calcul (13) ;
une unité de détection (14), configurée pour détecter les asynchronies (2) entre le respirateur (1) et l'être vivant sur la base de paramètres respiratoires de l'être vivant ;
une unité de mémoire (15) ;
une unité de surveillance (16) ;
une unité de ventilateur et/ou de valve (18) ; et
une unité de commande (17), configurée pour, au moins sur la base des asynchronies détectées par l'unité de détection, commander automatiquement, au moins partiellement et au moins temporairement, le respirateur (1) au moyen de la commande de l'unité de ventilateur et/ou de valve (18) ;

**caractérisé en ce que** l'unité de calcul (13) est configurée pour calculer une résistance des voies aériennes R et une élastance pulmonaire E à partir de valeurs de mesure acquises par l'unité de capteurs (11) et traitées par l'unité de traitement (12) au moyen d'un modèle pulmonaire mathématique, pour déterminer un débit respiratoire attendu (204, 304) à partir de la résistance des voies aériennes R et de l'élastance pulmonaire E, et pour déterminer un débit d'effort respiratoire (209, 306) en tant qu'écart du débit respiratoire attendu (204, 304) par rapport à un débit respiratoire déterminé (203, 303), le débit respiratoire déterminé (203, 303) représentant un débit respiratoire du patient calculé à partir d'un débit mesuré par le respirateur (1) après soustraction d'une fuite et d'autres influences, l'unité de détection (14) étant en outre configurée pour détecter des cycles respiratoires manqués (218) par évaluation du débit d'effort respiratoire (209, 306), du débit respiratoire attendu (204, 304) et du débit respiratoire déterminé (203, 303) et pour les évaluer comme étant les asynchronies (2), les cycles respiratoires manqués (218) étant ceux pour lesquels l'être vivant présente un effort respiratoire, mais l'effort respiratoire n'est pas détecté par le respirateur (1), de sorte qu'aucune assistance inspiratoire n'est fournie, l'unité de détection (14) détectant les cycles respiratoires manqués (218) sur la base d'au moins deux des caractéristiques de détection suivantes du débit d'effort respiratoire (209, 306), du débit respiratoire attendu (204, 304) et du débit respiratoire déterminé (203, 303) :

- le maximum local (206) du débit respiratoire déterminé (204) se situe entre deux minima (214, 215) du débit d'effort respiratoire (209, 306) ;
- Différence (210) entre la position temporelle du maximum local (219) du débit d'effort respiratoire (209, 306) et le minimum gauche correspondant (214) ;
- Différence (211) entre la position temporelle du maximum local (219) du débit d'effort respiratoire (209, 306) et le minimum droit correspondant (215) ;
- Différence (212) entre les valeurs du maximum local (219) du débit d'effort respiratoire (209, 306) et le minimum gauche correspondant (214) ;
- Différence (213) entre les valeurs du maximum local (219) du débit d'effort respiratoire (209, 306) et le minimum droit correspondant (215) ;
- Débit respiratoire attendu (207) au moment du maximum local (219) du débit d'effort respiratoire (209, 306) ;
- Temps entre le maximum local (219) du débit d'effort respiratoire (209, 306) et un instant de déclenchement attendu (205) du respirateur (1).

**2.** Système selon la revendication 1,
dans lequel l'unité de détection (14) détecte les cycles respiratoires manqués (218) sur la base d'au moins quatre caractéristiques de détection ou de l'ensemble des caractéristiques de détection.

**3.** Système selon l'une des revendications précédentes,
dans lequel l'unité de détection (14) est en outre configurée pour détecter de brefs retards de déclenchement (308) et/ou des déclenchements erronés (307) et/ou des déclenchements expiratoires prématurés (412) et pour les évaluer comme étant les asynchronies (2).

**4.** Système selon la revendication 3,
dans lequel l'unité de détection (14) est en outre configurée pour détecter les brefs retards de déclenchement (308) par l'évaluation du débit d'effort respiratoire (209, 306), du débit respiratoire attendu (204, 304) et du débit respiratoire déterminé (203, 303).

**5.** Système selon la revendication 3 ou 4,
dans lequel l'unité de commande (17) est configurée pour régler automatiquement une sensibilité de déclenchement (3) du respirateur (1) sur la base des cycles respiratoires manqués (218) et des brefs retards de déclenchement (308) détectés par l'unité de détection (14).

**6.** Système selon la revendication 5,
dans lequel l'unité de commande (17) est en outre configurée pour régler automatiquement la sensibilité de déclenchement (3) du respirateur (1) sur la base des déclenchements erronés (307) détectés par l'unité de détection (14).

**7.** Système selon la revendication 5 ou 6,
dans lequel l'unité de commande (17) est configurée pour adapter automatiquement la sensibilité de déclenchement (3) en fonction du nombre de brefs retards de déclenchement détectés (308) et de cycles respiratoires manqués (218) dans un intervalle de temps compris entre 0,5 et 5 minutes, de préférence entre 1 et 3 minutes.

**8.** Système selon l'une des revendications 5 à 7,
dans lequel , pour des débits de fuite supérieurs à une valeur seuil comprise entre 15 l/min et 50 l/min, en particulier de 25 l/min, la sensibilité de déclenchement (3) est réglée sous la forme d'une sensibilité de déclenchement moyenne qui est déterminée en tenant compte des sensibilités de déclenchement (3) issues de périodes antérieures présentant des débits de fuite inférieurs à la valeur seuil.

**9.** Système selon l'une des revendications 5 à 8,
dans lequel la sensibilité de déclenchement (3) est réglable manuellement et automatiquement, le réglage automatique de la sensibilité de déclenchement (3) pouvant régler des valeurs seuil des paramètres de déclenchement plus faibles que celles possibles par le réglage manuel.

**10.** Système selon l'une des revendications 5 à 9,
dans lequel la sensibilité de déclenchement (3) comprend une valeur qui commande la commutation d'une phase d'inspiration vers une phase d'expiration, le respirateur(1) étant configuré pour régler, sur la base des déclenchements d'expiration prématurés (412) détectés, la valeur de la sensibilité de déclenchement (3) commandant la commutation d'une phase d'inspiration vers une phase d'expiration.

**11.** Système selon l'une des revendications 3 à 10,
dans lequel un retard de déclenchement (305) est détecté en tant que bref retard de déclenchement (308) lorsque ledit retard de déclenchement (305, 308) est inférieur ou égal à une valeur seuil, ladite valeur seuil étant choisie dans une plage comprise entre 0 seconde et 0,5 seconde, de préférence entre 0 seconde et 0,25 seconde, de façon particulièrement préférée entre 0 seconde et 0,15 seconde.

**12.** Système selon la revendication 11,
dans lequel l'unité de détection (14) est configurée pour détecter et déterminer le retard de déclenchement (305) par l'intermédiaire d'un décalage entre l'effort respiratoire de l'être vivant et le déclenchement du respirateur (1).

**13.** Système selon l'une des revendications 3 à 12,
dans lequel le respirateur (1) est configuré pour détecter les déclenchements expiratoires prématurés (412) sur la base d'une évolution temporelle du débit respiratoire déterminé (403) et/ou du débit respiratoire attendu (404) et/ou du débit d'effort respiratoire (409).

**14.** Système selon l'une des revendications 3 à 13,
dans lequel le respirateur (1) est configuré pour détecter les déclenchements expiratoires prématurés (412) sur la base de la position et de la valeur de débit associées à un début (405) de rampe de pression, à une fin (406) de rampe de pression, à un maximum local (407, 410) et/ou à un minimum local (408) du débit respiratoire déterminé (403) et/ou du débit respiratoire attendu (404).

**15.** Système selon l'une des revendications 3 à 14,

dans lequel le respirateur (1) est configuré pour détecter les déclenchements expiratoires prématurés (412) lorsque au moins l'une des conditions suivantes est remplie :

- le débit respiratoire déterminé (403) est supérieur à 0 l/min au début (405) d'une rampe de pression ;
- le débit respiratoire déterminé (403) est inférieur à 0 l/min à la fin (405) d'une rampe de pression ;
- e * débit respiratoire déterminé (403) au maximum local (407) / débit respiratoire déterminé (403) à la fin (406) d'une rampe de pression < débit respiratoire attendu (404) / débit respiratoire attendu (403) à la fin de la rampe de pression ;
- f * débit respiratoire déterminé (403) au maximum local (407) > débit respiratoire déterminé (403) à la fin (406) d'une rampe de pression ;
- g * débit respiratoire déterminé (403) au minimum local (408) < débit respiratoire déterminé (403) à la fin (406) d'une rampe de pression + débit respiratoire déterminé (403) au maximum local (407) ;
- (h < débit respiratoire déterminé (403) au maximum local (407) - débit respiratoire déterminé (403) à la fin (406) de la rampe de pression) OU (h < débit respiratoire déterminé (403) au maximum local (407) - débit respiratoire déterminé (403) au minimum local (408)) ;
- débit respiratoire déterminé (403) au minimum local (408) < 0 ;

dans lequel le facteur e est compris dans une plage allant de 0,5 à 2,0, de préférence de 1 à 1,8, dans lequel le

facteur f est compris dans une plage allant de 0,9 à 2,9, de préférence de 1,7 à 2,3, dans lequel le facteur g est compris dans une plage allant de 1 à 3, de préférence de 1,8 à 2,2, et dans lequel h est compris dans une plage allant de 2 l/min à 10 l/min, de préférence de 3 l/min à 7 l/min.

**16.** Système selon l'une des revendications précédentes, dans lequel la résistance des voies aériennes R et l'élastance pulmonaire E sont déterminées par l'unité de calcul (13) au moyen d'une régression linéaire multiple et du modèle pulmonaire à un compartiment.

1
11
15
12
16
13
17
14
18

Figur 1

216
217
218
60
40
20
0
-20
-40
-60
201
204
203
205
206
207
214
215
208
0
1
2
3
4
5
6
7
8
202

Figur 2

10
8
6
4
2
0
-2
-4
-6
-8
0
1
2
3
4
5
6
7
8
201
202
209
210
219
211
212
213
214
215

Figur 3

309
309
309
80
60
40
20
0
-20
-40
-60
301
304
305
305
305
305
303
0
1
2
3
4
5
6
7
8
302

Figur 4

309
309
309
305
305
305
306
15
10
5
0
-5
-10
-15
-20
301
0
1
2
3
4
5
6
7
8
302

Figur 5

411
411
100
80
60
40
20
0
-20
-40
-60
0
1
2
3
4
5
6
7
8
401
402
403
404
405
406
407
408

**Figur 6**

411
411
40
30
20
10
0
-10
-20
-30
410
409
401
0
1
2
3
4
5
6
7
8
402


Figur 7

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 2016325061 A1